# EUROPEAN PATENT APPLICATION

(11) **EP 0 683 228 A2**
(43) Date of publication of application: **22.11.1995**
(21) Application number: 95110254.0
(22) Date of filing: 01.02.1988
(51) Int. Cl.: C12N 15/60, C12N 15/62, C12N 15/80, C12N 9/88, C12N 1/14, C12N 1/15, C12N 1/20

(54) **Novel expression system**

(30) Priority: 04.02.1987 GB 8702475
(62) Divisional of application: 88101397.3
(71) Applicant: CIBA-GEIGY AG, CH-4002 Basel (CH)
(72) Inventor: Heim, Jutta, Dr., CH-4133 Pratteln (CH); Gysler, Christof, Dr., CH-1807 Blonay (CH); Visser, Jacob, Prof., NL-6703 CK Wageningen (NL); Kester, Hermanus Cornelis Maria, NL-6651 DR Druten (NL)
(74) Representative: Zumstein, Fritz, Dr.

(57) **Abstract**

Recombinant DNA molecules coding for a pectin lyase expression system and derivatives thereof, such as the structural gene of PLI and corresponding regulatory sequences, e.g. promoter, signal and terminator sequences, and hybrid vectors comprising corresponding DNAs, including hybrid vectors with DNA coding for homologous or heterologous polypeptides, hosts, especially filamentous fungi, e.g. Aspergillus hosts, transformed by said vectors, methods for the preparation of said recombinant DNA molecules and said hosts and the use of the recombinant DNA molecules for the preparation of new expression systems. A further objective is the preparation of polypeptides by means of said DNAs and said hosts.

## Description

### Field of the invention

The invention relates to the field of genetic engineering and provides novel DNA molecules comprising DNA sequences coding for the pectin lyase I (PLI) of Aspergillus niger and/or the promoter, signal sequence or terminator thereof. The novel DNA molecules are useful for the overproduction of PLI in Aspergillus and/or the construction of hybrid vectors expressing foreign genes in filamentous fungi.

### Background of the invention

Although in genetic engineering techniques numerous polypeptide expression systems for prokaryotic and eukaryotic hosts are already known, there is a continuous need for novel systems which may have advantages over the known systems.

Very widely used are the prokaryotic Escherichia coli host and the eukaryotic yeast host, e.g. Saccharomyces cerevisiae, for which a high number of different expression hybrid vectors, mostly plasmids, have been developed. The drawbacks of E. coli hosts are that they cannot glycosylate the formed polypeptide and that for lack of secretion the foreign peptide may accumulate within the host cell and prevent further growth. The yeast hosts do glycosylate, however, like E. coli, they do not secrete the polypeptides, except very small ones, into the nutrient medium. Yeasts secrete only into the periplasmic space. Higher eukaryotic hosts are mammalian cancer cells which are able to glycosylate and secrete into the nutrient medium, however, cultivation thereof is very slow and expensive and the danger exists that oncogenic nucleic acids are isolated together with the desired peptide of which the latter may not be freed.

In the need for other hosts also filamentous fungi, such as Neurospora crassa, Aspergillus nidulans and Aspergillus niger, have been investigated. Such fungi are already widely used for industrial purposes, however, the application thereof in genetic engineering techniques has lagged behind, mainly for lack of an appropriate transformation system. In contrast to Saccharomyces cerevisiae, filamentous fungi do not contain plasmids which could be used for the introduction of foreign genes and phenotype selection. It is, however, possible to transform filamentous fungi with foreign plasmids containing a selectable marker gene. All vectors described so far for filamentous fungi do not autonomously replicate, as these of yeasts do, but are integrated into the fungal chromosome. This event occurs only at a very low frequency. Advantageously, on the other hand, integrative transformation renders the transformants mitotically very stable, even under non-selective conditions. Stable integration of more than one hundred copies has been reported.

The first vector for filamentous fungi described contained the qa-2 gene of Neurospora crassa as selectable marker. This gene encodes the enzyme catabolic dehydraquinase and can be used for functional complementation of aro mutants of N. crassa [Case, M.E., Schweizer, M., Kushner, S.R. and Giles, N.H. (1979) Proc. Natl. Acad. Sci. USA 76, 5259-5263]. Aro mutants are unable to grow on minimal medium without an aromatic amino acid supplement. Transformation of N. crassa by the qa-2 vector occurred by integration of a single copy of the plasmid into the chromosome. 30 % of stable aro⁺ integrants retained the integrated qa-2 gene still linked to the bacterial plasmid sequences [Case, M.E. (1982) in Genetic Engineering of Microorganisms for Chemicals (Holländer, A., DeMoss, D., Kaplan, S., Kanisky, J., Savage, D. and Wolfe, R.S., eds), pp. 87-100, Plenum]. This observation made cotransformation of non-selective DNA-sequences together with selective ones a feasible task.

In Aspergillus nidulans, which has a sexual cycle and is therefore amenable to classical genetic manipulations, both negative and positive selection systems have been identified. Either using heterologous DNA from N. crassa or homologous DNA, functional complementation of A. nidulans pyrG-mutants by transformation with plasmids containing the pyrG gene was obtained (Ballance et al. BBRC 112, 284 1983; Tilburn et al. Gene 26, 205, 1983). In other Systems mutations at the trpC or argB locus were functionally complemented by transformation with the appropriate plasmids [Yelton et al. PNAS 81, 1470, 1984; Yelton et Timberlake J. Cell. Bio-chem. Suppl. 9C 173, 1985; Johnstone et al. EMBO J. 4, 1307, 1983].

A dominant positive selection system has also been developed making use of the amdS gene isolated from A. nidulans which enables A. niger transformed therewith to grow on acetamide as sole nitrogen source (Tilburn et al., Gene 26, 205, 1983); Wernars et al., Curr.Genet. 9, 361, 1985, Kelly, J.M. et al., EMBO J. 4, 475,1985).

Compared to N. crassa or A. nidulans, A. niger is by far the more important organism. It is used widely in the industrial production of enzymes, e.g. for use in the food industry. A. niger differs from A. nidulans by its secretory capacity, in that it secretes a variety of hydrolytic enzymes, e.g. glucoamylase, α-amylase, pectinase, cellulase, β-glucanase, β-galactosidase, naringinase, pentosanase, acid protease and lignase, the glucoamylase and pectinase complex being the most important ones.

A. niger has no known sexual cycle. Mutations can therefore not be introduced via meiotic recombinations. By classical mutation and selection procedures, extensive strain improvements in the secretion of hydrolytic enzymes have however been achieved.

Of the genes of A. niger enzymes only those of glucoamylase (Boel et al. EMBO J. 3, 1581, 1984) and alcohol and aldehyde dehydrogenase (WO 86/06097) together with their promoter and signal sequences have been characterised and used in transformation experiments with A. nidulans and A. niger, respectively.

As selection markers for A. niger have been used the heterologous amds gene (Kelly and Hynes, EMBO 3. 4, 475, 1985), and the argB gene (Buxton et al., Gene 37, 207, 1985; EP 184 438; WO 86/06097), both obtained from A. nidulans.

A. niger is the most important organism for the industrial production of pectin degrading enzymes. Pectins are polygalacturonides of high molecular weight (20000-40000 D) consisting of α-1,4-glycosidic bounded D-galacturonic acid polymers and occur in nature as constituents of higher plant cells, where they are attached to cellulose molecules mainly found in the primary cell wall and the middle lamella. Amongst the richest sources of pectin are lemon and orange rind, which contain about 30 % of this polysaccharide. Pectic enzymes are degrading the carbohydrate polymer substrate either by hydrolysis of the α-1,4-glycosidic bond (polygalacturonase) or by transelemination of the α-4,5 unsaturated galacturonic residue from the pectin molecule (different pectin lyases). The systematic name of pectin lyase is pectin transeliminase (EC 4.2.2.10).

In A. niger the proteins of the pectic complex are not expressed constitutively. Under inducing conditions using pectin or breakdown products thereof A. niger expresses the above mentioned enzymes, including PLI, when other carbon sources, such as glucose or sucrose, are limiting. In surface cultures the pectic enzymes tend to remain associated with the outer cell wall. Increasing pectin and Ca²⁺ concentration in the medium leads to complete secretion.

Pectinases, such as PLI, are used by the food stuff industry mainly for fruit juice clarification.

From A. niger two different pectin lyases, PLI and PLII, have been purified and partially characterized by F.E.A. Van Houdenhoven (1). PLI contains four residues of mannose, whereas PLII has two residues of mannose and glucose each. The enzymes have different molecular weights (PLI: 37.5 kD, PLII: 36 kD). Before the present invention no total or partial amino acid sequences have been published.

The present invention is based on a partial structure determination of pectin lyase I (PLI) which allowed the synthesis of DNA probes coding for relevant parts of the protein. By means of the DNA probes it was possible to screen for and isolate DNA coding for PLI, eventually together with pre- and post-sequences thereof, from a gene library of A. niger.

### Objects of the invention

Objects of the invention are recombinant DNA molecules coding for a pectin lyase expression system and derivatives thereof, such as the structural gene of PLI and corresponding regulatory sequences, e.g. promoter, signal and terminator sequences, and hybrid vectors comprising corresponding DNAs, including hybrid vectors with DNA coding for homologous or heterologous polypeptides, hosts, especially filamentous fungi, e.g. Aspergillus hosts, transformed by said vectors, methods for the preparation of said recombinant DNA molecules and said hosts and the use of the recombinant DNA molecules for the preparation of new expression systems. A further objective is the preparation of polypeptides by means of said DNAs and said hosts.

The pectin lyase expression system comprises DNA sequences coding for the promoter, the signal sequence, the structural gene and the terminator of a pectin lyase gene.

More particularly, one object of the present invention is the construction of hybrid expression vectors comprising DNA sequences coding for the promotor of PLI, optionally for the signal sequence of this protein, and optionally for a suitable terminator of the same gene. These hybrid expression vectors are used for the incorporation of genes coding for particular proteins and for the transformation of filamentous fungi, such as Aspergillus, Penicillium and Cephalosporium. Cotransformation of A. niger with 2 different plasmids can be carried out, whereby one of the plasmids is selected from the group of the expression vectors of the invention, and the other one is a specially constructed selection plasmid, which works in connection with a mutated strain of a filamentous fungus.

The present invention also concerns the overproduction of PLI in Aspergillus species.

The various subjects of the invention will become more evident from the following detailed description of the invention.

### Detailed description of the invention

### The Recombinant DNA Molecules

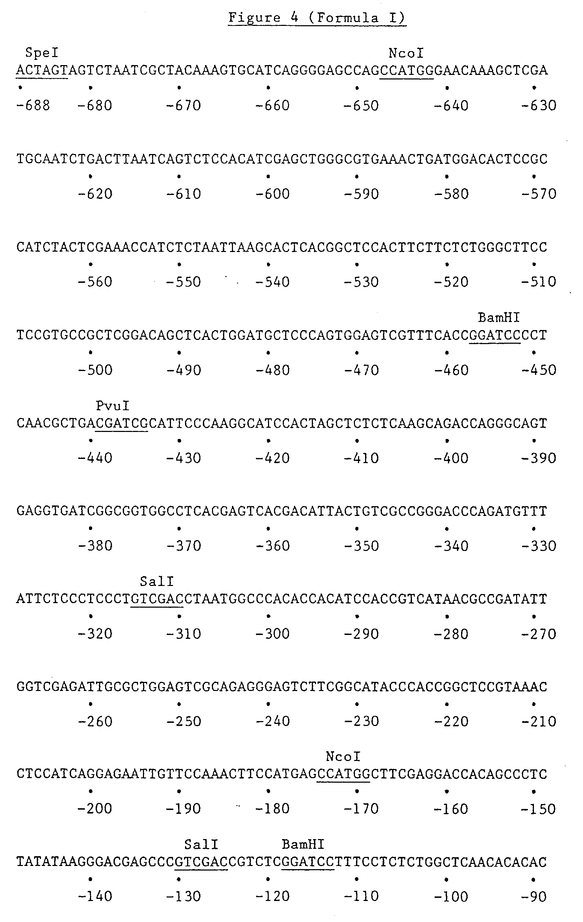

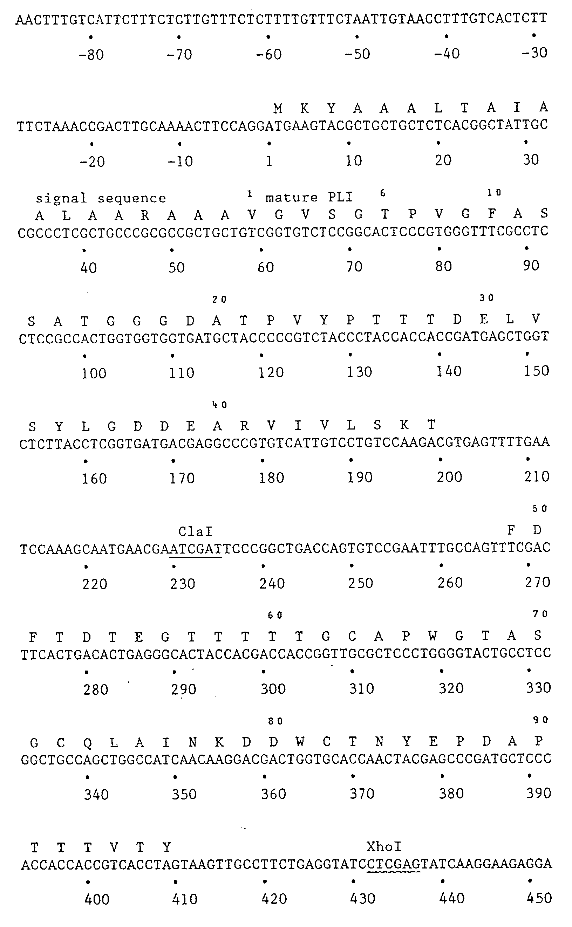

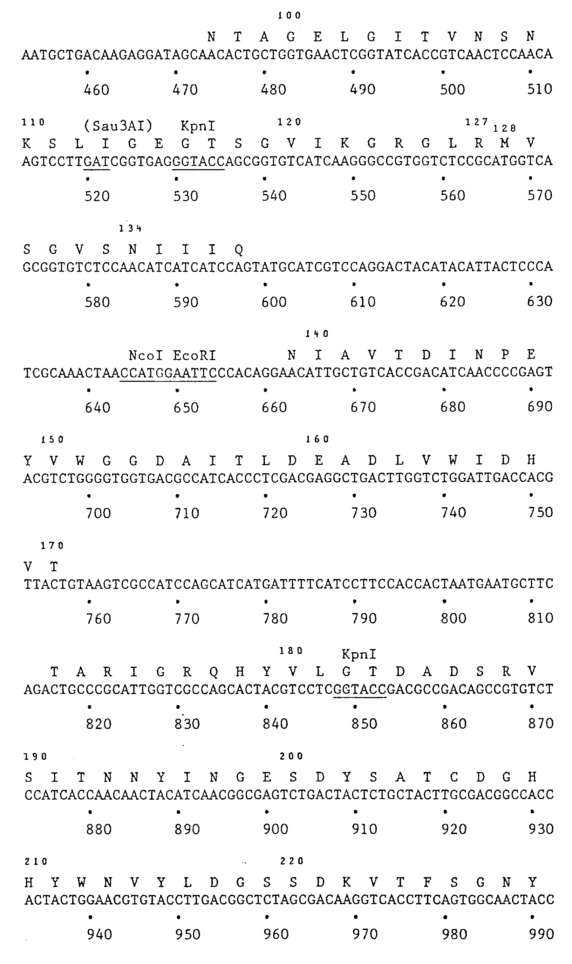

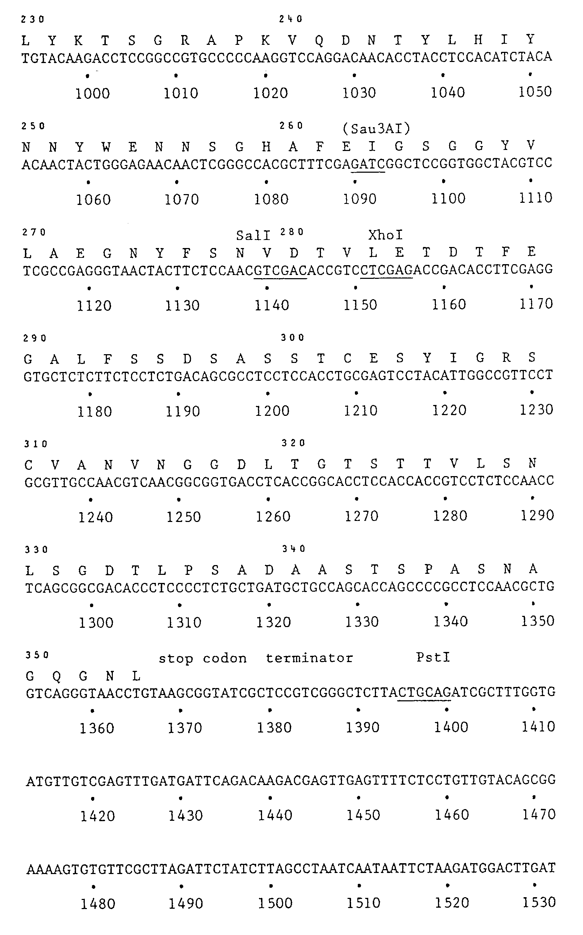

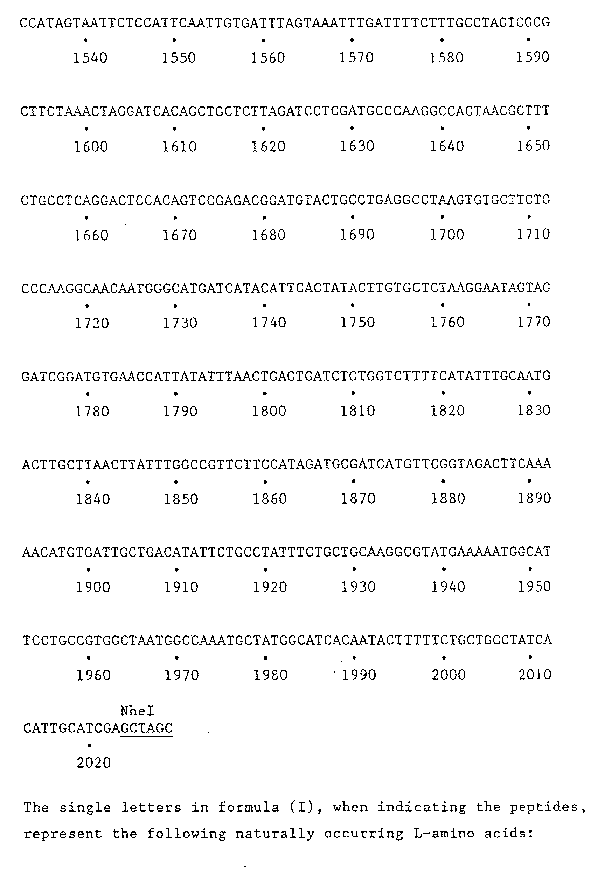

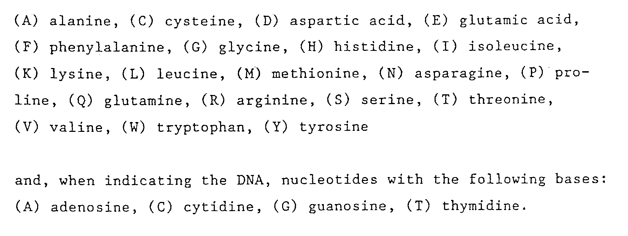

More particularly, the present invention concerns a recombinant DNA molecule comprising the DNA sequence of the formula I (Figure 4) or a derivative thereof.

The term "derivative" when used in connection with the DNA sequence of the formula I is intended to include larger derivatives with flanking sequences, fragments of said DNA sequence, mutants, especially naturally occurring mutants, and DNA sequences which are degenerated.

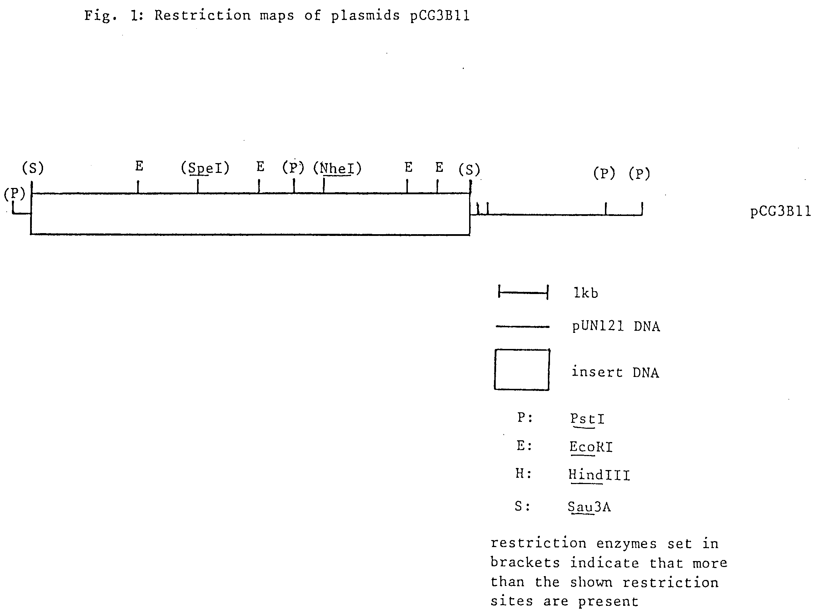

Larger derivatives of the DNA molecules of the formula I are those excisable from the A. niger genome and comprising the DNA sequence of the formula I, such as can be found in a genomic library of A. niger obtained by fragmentation of the nucleic acids, treatment of the fragments with a suitable restriction enzyme, e.g. Sau3A, ligating into a suitable plasmid, e.g. pUN121, cloning, e.g. in E. coli, and excising again, with the same restriction enzyme. Such derivative is e.g. the insert of plasmid pCG3B11 shown in Figure 1.

Fragments of the DNA sequence of the formula I are those extending between two restriction sites selected from those of Pst I, EcoRI, HindIII, Sau3AI, KpnI, ClaI, NcoI, AccI, SalII, PvuI, SalI, SpeI, BglI, EcoRV, BglII, AvaI, XhoI, NheI, BssHI, XmnI, BamHI, BssHII, and the like, as shown in the accompanying Figures.

Preferred fragments are those containing the promoter sequence, the signal sequence, the structural gene of PLI, and/or the terminator sequence.

The fragments of the DNA sequence of the formula I may contain linkers which provide for successful linkage to other DNA molecules.

The PLI promoter sequence is contained in the DNA region between nucleotide positions -1 to -688, whereby the sequence between nucleotides about -100 and -146 is essential. For the promoter sequence the TATAA box at nucleotides -120 to -146 is important as the RNA-polymerase recognition site. Suitable linkers may be attached to these fragments.

The promoter of PLI of A. niger is inducible, i.e., the expression of the structural gene attached thereto, e.g. the structural gene coding for PLI or any other foreign gene, is induced by addition of pectin or pectin degradation products to the medium.

The signal sequence extends between nucleotides 1 to 57. The signal sequence is a preferred sequence and DNA molecules comprising it, e.g. such containing said sequence and optional suitable linkers, are preferred DNA molecules.

The structural gene of PLI starts at nucleotide 58 and extends to nucleotide 1366. As is evident from formula I the structural gene contains several introns. Also the structural gene may contain suitable linkers.

The terminator starts with the stop codon TAA at nucleotide 1367 and may extend up to nucleotide 1570 or up to 2029, especially up to one of the restriction sites within said sequence. The terminator fragment may contain suitable linkers.

Suitable linkers to above fragments have a DNA sequence which fits into the restriction site of the DNA to which the fragment is to be linked, or which contain a restriction site.

Fragments of the DNA sequence of the formula I are also such which are composed of smaller fragments, e.g. those composed of the promoter and the signal or structural sequence, or of the signal and the structural sequence, the structural gene without the introns, and the like.

Mutants of the DNA sequence of the formula I are e.g. naturally occurring mutants, such as the mutant whose nucleotide T₈₀ is replaced by A₈₀, whereby the codon GTG coding for valine is altered to GAG coding for glutamic acid. The latter amino acid is present in the PLI isolated from a mixture of pectinolytic enzymes (Ultrazym®, Nova Industries, Copenhagen) obtained from an A. niger strain. The invention comprises also natural or synthetic mutants of the signal sequence with a similar or identical hydrophobicity profile, e.g. wherein the codons for the polar amino acids lysine (K^{δ+}), tyrosine (Y^{δ-}) and arginine (R^{δ+}) are exchanged by codons for other amino acids having similar charges, and the hydrophobic amino acids alanine (A), leucine (L) and threonine (T), are replaced by codons for other hydrophobic amino acids. For example, the codon for the positively charged lysine may be replaced by a codon far arginine and vice versa, the codon for the negatively charged tyrosine by a codon for glutamate or aspartate, and/or the codon for the non-polar, hydrophobic alanine by any one of the codons for threonine, proline, valine, isoleucine, leucine, methionine or phenylalanine, and the like. Other mutations are "silent mutations" wherein one or a few other nucleotides, e.g. up to about 30, are replaced by one or more other nucleotides, whereby the new codons code for the same amino acid(s).

DNA sequences of the formula I which are degenerated are, like silent mutations, such which are degenerated within the meaning of the genetic code in that an unlimited number of nucleotides are replaced by other nucleotides without changing the amino acid sequence for which they code. Such degenerate DNA sequences may be useful for their different restriction sites.

Recombinant DNA molecules comprising a DNA sequence of the formula I or a derivative thereof are especially recombinant vectors, alternatively called hybrid vectors, containing such DNA sequences as inserts. They are usable for cloning in hosts, such as bacteria, fungi or animal cells. Such hybrid vectors are derived from any vector useful in the art of genetic engineering, such as from phages, cosmids, plasmids or chromosomal DNA, such as derivatives of phage λ, e.g. NM 989, M13mp8 phage DNA(15) linearized by BamHI digestion, bacterial plasmids, e.g. pBR 322, pUN121, pUC18, or yeast plasmids, e.g. yeast 2µ plasmid, or also chromosomal DNA, derived e.g. from Aspergillus, e.g. A. niger, for example those provided by EP 184 438.

A hybrid vector of the invention, in addition to the DNA sequence of the formula I or a derivative thereof, contains a replication site and optional, depending on the type of the DNA derivative, an expression control sequence, such as an enhancer sequence, upstream activation site, promoter and signal sequences, and/or structural genes different from corresponding present A. niger derived sequences. Such enhancer sequences may be derived from the extrachromosomal ribosomal DNA of Physarum polycephalum (PCT/EP 8500278), or are the upstream activation sites from the acid phosphatase PHO5 gene (EP Appl. No. 86 111 820.6), or the PHO5, trp, PHO5-GAPDH hybrid (EP Appl. No. 86 111 820.6), or the like promoters. Such expression control sequences may be linked to the PLI structural gene.

Structural genes ligated to the present promoter, signal and/or terminator sequences are, besides those coding for PLI with or without introns, also homologous other pectin lyase genes, and heterologous structural genes coding for a wide variety of polypeptides, including glycosylated polypeptides, in particular of higher eukaryotic, especially mammalian, such as animal or especially human origin, such as enzymes which can be used, for example, for the production of nutrients and for performing enzymatic reactions in chemistry, or non-enzymatic polypeptides, which are useful and valuable for the treatment of human and animal diseases or for the prevention thereof, far example hormones, polypeptides with immunomodulatory, anti-viral and anti-tumor properties, antibodies, viral antigens, vaccines, clotting factors, foodstuffs and the like.

Examples of such heterologous structural genes are e.g. those coding for insulin, growth factors, such as epidermal, insulin-like, mast cell, nerve or transforming growth factor, growth hormones, such as human or bovine growth hormones, interleukin, such as interleukin-1 or -2, human macrophage migration inhibitory factor (MIF), interferons, such as human α-interferon, for example interferon-αA, αB, αD or αF, β-interferon, γ-interferon or a hybrid interferon, for example an αA-αD- or an αB-αD-hybrid interferon, hepatitis virus antigens, such as hepatitis B virus surface or core antigen or hepatitis A virus antigen, plasminogen activators, such as tissue plasminogen activator or urokinase, tumour necrosis factor, somato-statin, renin, β-endorphin, immunoglobulins, such as the light and/or heavy chains of immunoglobulin D, E or G, immunoglobulin binding factors, such as immunoglobulin E binding factor, calcitonin, human calcitonin-related peptide, blood clotting factors, such as factor IX or VIIIc, eglin, such as eglin C, desulfatohirudin, such as desulfatohirudin variant HV1, HV2 or PA, or human superoxide dismutase. Preferred genes are those coding for a human α-interferon or hybrid interferon, human tissue plasminogen activator (t-PA), hepatitis B Virus surface antigen (HBVsAg), insulin-like growth factor I, eglin C and desulfatohirudin, e.g. variant HV1. In the hybrid vectors of the present invention, the present promoter and/or signal sequence is operably linked to the polypeptide coding region so as to ensure effective expression of the polypeptide.

The DNA molecules of the present invention may contain selective markers depending on the host which is to be transformed, selected and cloned. Any marker gene can be used which facilitates the selection of transformants due to the phenotypic expression of the marker. Suitable markers are particularly those expressing antibiotic resistance, e.g. against tetracycline or ampicillin, or, in the case of auxotrophic yeast mutants, genes which complement host lesions. Corresponding genes confer, for example, resistance to the antibiotic cycloheximide, or provide for prototrophy in an auxotrophic yeast mutant, for example the ura3, leu2, his3 or trp1 gene. It is also possible to employ as markers structural genes which are associated with an autonomously replicating segment providing that the host to be transformed is auxotrophic for the product expressed by the marker.

Of particular importance are marker genes which complement A. niger host lesions, such as the argB gene coding for the ornithine carbamoyl transferase, e.g. derived from A. niger or A. nidulans (EP 184 438), or A. nidulans DNA fragments homologous to the N. crassa pyr4 gene (26).

Preferred embodiments of the present invention are hybrid vectors wherein the structural gene, especially the foreign structural gene is operatively linked to the promoter and signal sequences of the present invention. Such preferred hybrid vectors are e.g. pCG3B11, M13 mp8-PL (SalI-EcoRI) BssHII/BE5, pUBE5, pLHK3, pLHL5 and pLHLT7. Further useful hybrid vectors are pPL29-5 and pPL35-5 (see Examples and Figures).

The DNA of the formula I and the derivatives thereof, including fragments can be used for screening DNA gene banks or mRNA for similar DNAs or mRNAs.

### Process for the Preparation of the Recombinant DNA Molecules

The invention concerns also a process for the preparation of a recombinant DNA molecule coding for a pectin lyase expression system or a derivative thereof, comprising culturing a host transformed with a DNA molecule containing a DNA sequence coding for the pectin lyase expression system or a derivative thereof and isolating the desired recombinant DNA molecule or the derivative thereof, or preparing it by an in vitro synthesis.

The culturing of the hosts ist carried out in a conventional nutrient medium which may be supplemented with or deprived of chemical compounds allowing negative or positive selection of the transformants, i.e. such hosts containing the desired DNA molecule together with a selection marker, from the non-transformants, i.e. such hosts lacking the desired DNA molecule.

Any transformable hosts useful in the art may be used, e.g. bacteria, such as E. coli, fungi, such as Saccharomyces cerevisiae, or in particular filamentous fungi, such as Aspergillus, e.g. A. nidulans, A. oryzae, A. carbonarius, A. awamori and especially A. niger. A preferred host is A. niger An8, a novel mutant lacking the pyrA gene, as described further below. Transformation of the hosts is carried out by conventional methods.

The DNA sequence coding for the PL expression system is obtained from a filamentous fungi containing such system, in particular from a genomic library thereof or also via the mRNA. In the following the preparation of the PLI expression system is described in more detail.

A genomic library can be prepared e.g. by partial digestion of genomic DNA of an A. niger strain, e.g. N756, with Sau3AI and cloning the high molecular weight DNA fragments into the E. coli plasmid pUN121. Any other A. niger strain producing PLI may serve as source for the genomic library and Likewise other suitable vectors may be used as recipient for the fragments.

In order to successfully screen the genomic library for DNA sequences coding for PLI, sequence determination of this enzyme or parts thereof was necessary. PLI was purified from a commercially available mixture of pectinolytic enzymes of A. niger (Ultrazym®, Novo Industries) and sequenced. The N-terminus of mature PLI revealed the sequence
V₁-G-V-X₄-G-T₆-P-E-G-F-A,
and a fragment of PLI, obtained by splitting of PLI with cyanogen bromide, revealed the N-terminal sequence
V₁₂₉-S-G-V-S-N-I-I-I-Q-N-I-A-V-X₁₄₃-D-I-N-X₁₄₇-E,
wherein the amino acids X at that time had not been identified.

Based an those amino acid sequences a number of DNA probes, i.e. mixtures of DNA sequences coding for parts of the amino acid sequences and the corresponding degenerate DNA sequences, were chemically synthesized and used for screening. Such DNA probes are for example the mixtures of the formulas
3' TGR₁ GGR₁ CTR₂ CCR₃ AAR₃ CG5' (2014) (Ia) or
3' TGR₁ GGR₁ CTR₂ CCR₂ AAR₃ CG5' (2015) (Ib)
wherein R₁ is A,C, G or T, R₂ is C or T and R₃ is A or G, or DNA mixture 2060 of the formula
5' AAR₁ ATR₂ ATR₂ ATR₂ CAR₃ A 3' (2060) (Ic)
wherein R₁ is A or T, R₂ is A, T or C and R₃ is A or G.

Since the total DNA sequence of the PLI gene became known during the course of this work, any part thereof having at least about 14 bp can now he used for screening, which includes also the screening for mRNA coding for the PL system.

For screening purposes the DNA probes are 5' radioactively labelled by methods known in the art using γ³²P-ATP and T4 kinase. Host microorganisms carrying nucleic acids of the present invention as an insert, are identified by hybridisation with the labelled DNA probe on filter replicas of the gene library.

For establishing a sublibrary, clones of the library, showing a radioactive response to the radioactive labelled DNA probe coding for the N-terminal part of PLI can be isolated, cultivated and hybridized with the second DNA probe coding for the N-terminal amino acids of the CNBr fragment, as described before.

Clones showing a hybridisation response to one or both DNA probes are isolated and amplified.

For example, screening with DNA probes 2014 and 2015 revealed 33 and 39 positive clones, respectively. The 72 clones were amplified and the obtained sublibraries rescreened with DNA probe 2060 whereby 3 positive clones could be identified. One clone was selected and named E. coli BJ 5183/pCG3B11. It contains plasmid pCG3B11 of which a partial restriction map is shown in Figure 1. The Sau3AI fragment of pCG3B11 comprises the SpeI-NheI fragment shown in Figure 4 and formula I.

Plasmid pCG3B11 can be used for constructing other DNA molecules of the invention by applying conventional genetic engineering techniques.

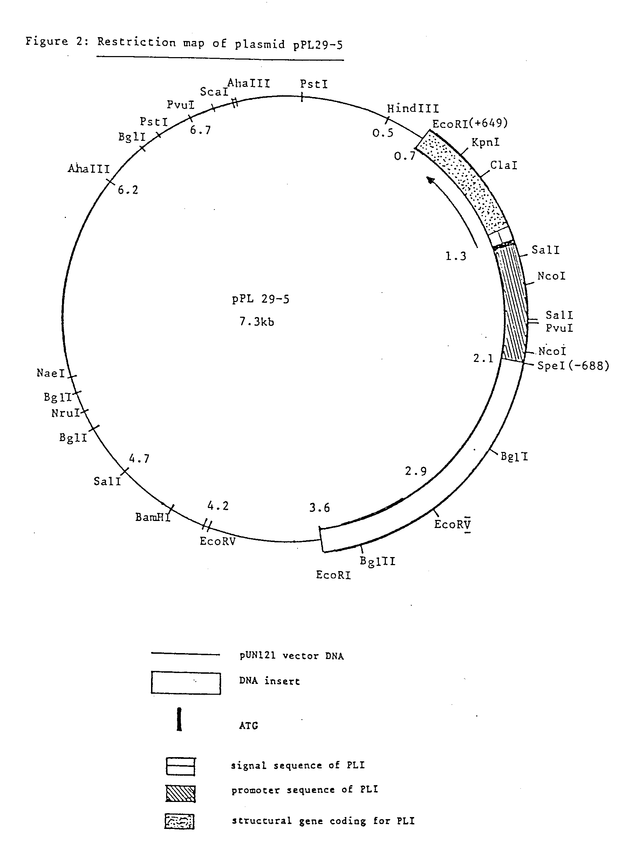

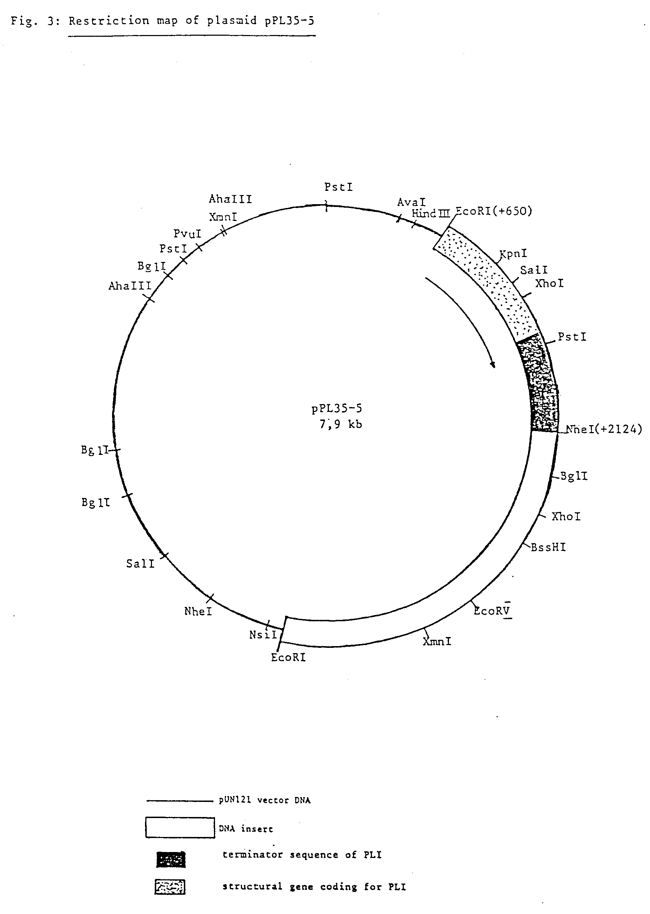

For example, restriction of pCG3B11 with EcoRI and cloning the two fragments into plasmid pUN121 leads to plasmid pPL 29-5 (Fig. 2) containing the promoter, the signal sequence and the part of the structural PLI gene up to the central EcoRI restriction site at position 649/650, and to plasmid pPL35-5 (Fig. 3) containing part of the PLI structural gene starting at position 650 and the terminator. pPL 29-5 contains further the flanking N-terminal sequences and pPL35-5 the flanking C-terminal sequences of pCG3B11.

Fragments of the inserts of plasmids pPL29-5 and pPL35-5 can be obtained for sequencing by restriction with suitable enzymes, such as AhaIII, PstI, AvaI, HindIII, EcoRI, KpnI, ClaI, NcoI, AccI, SalI, PvuI, SalII, NheI, BglI, EcoRI and EcoRV. The fragments can be subcloned, e.g. into different M13 phages, preferably M13mp8 and M13mp18, and sequenced, e.g. using the M13 Cloning and Sequencing Kit (M13 Sequencing Kit N.4502, Amersham) under the conditions given by the supplier (16). The entire sequence of the 2.7 kb SpeI-NheI fragment, comprising the complete sequence of PLI gene, is shown in Figure 4. It comprises 688 nucleotides of the promoter region, 1369 nucleotides of the structural part of the PLI gene and 661 nucleotides of the terminator region. The sequence of pPL29-5, corresponding to amino acid sequence of the N-terminus of PLI as determined by amino acid sequence analysis (Example 1), is preceded by 57 nucleotides coding for the signal peptide of the formula
M₁-K-Y-A-A-A-L-T-A-I-A-A-L-A-A-R-A-A-A₅₇
The amino acid sequence of the N-terminus of the C-terminal CNBr fragment of PLI (Example 3) is not continuously encoded by the cloned DNA fragment of pPL35-5 (nucleotide 1257-1379). A computer aided search for consensus sequences of exon/intron splice junctions as well as for intron internal sequences (Boel E. et al. (18) and Mount S.M. (19)) leads up to postulate the presence of four introns with length of 65 bp, 62 bp, 63 bp and 57 bp respectively (Fig. 4).

One phage, obtained from phages M13mp8 and the 0.8 kb SalI-EcoRI fragment of plasmid pPL29-5, containing 130 nucleotides of the promoter and the N-terminal part of the PLI gene, is designated M13mp8(SalI-EcoRI), and is used in further steps for the preparation of other DNA molecules of the invention.

Such other DNA molecules of the invention comprising fragments of the DNA sequence of the formula I can be constructed as described in Figures 5 to 9, whereby, when desired, novel restriction sites can be introduced, e.g. an BssHII restriction site within the signal sequence of the PLI gene. Such DNA molecules are the plasmids or phages, respectively, pUBE5, MBmp8-PL(SalI-EcoRI)BssHII/BE5 (Fig. 5), pLHK3 (Fig. 7), pLHL5, M13mp18-PL(SpeI-EcoRI)BssHII/AC5 pLHLT7 (Fig. 9), of which the latter three plasmids contain the structural gene coding for desulfatohirudin.

Mutants containing new restriction sites can be prepared, for example in vitro by site-directed mutagenesis, according to conventional methods [see review article of M.J. Zoller and M. Smith, Methods Enzymol. 100, 468 (1983), D. Botstein and D. Shortle, Science 229, 1193 (1985) or K. Norris et al., Nucl. Acids Res. 11, 5103 (1983)].

For example, a mutant of the present DNA sequences is derived from the M13mp8PL(Sal-EcoRI) phage which comprises the promoter and the secretional signal gene of PLI. Introduction of a new BssHII site into the signal sequence region of M13mp8PL(Sal-EcoRI) gene is performed by using a chemically synthesized primer oligonucleotide, which is complementary to the DNA sequence at positions 36 - 54 situated near the C-terminal part of the PLI signal sequence, with the exception that at position 45 a C/G transversion (mutagenic primer) is introduced.

The mutated phage, carrying the new BssHII site is designated as M13mp8PL (Sal-EcoRI) BssHII and can be used for the construction of expression vectors for homologous or heterologous genes.

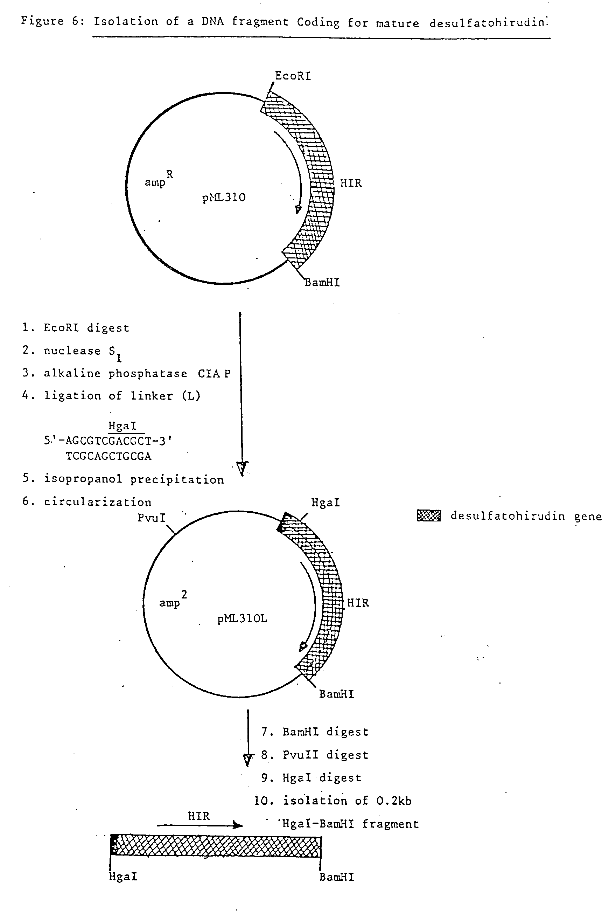

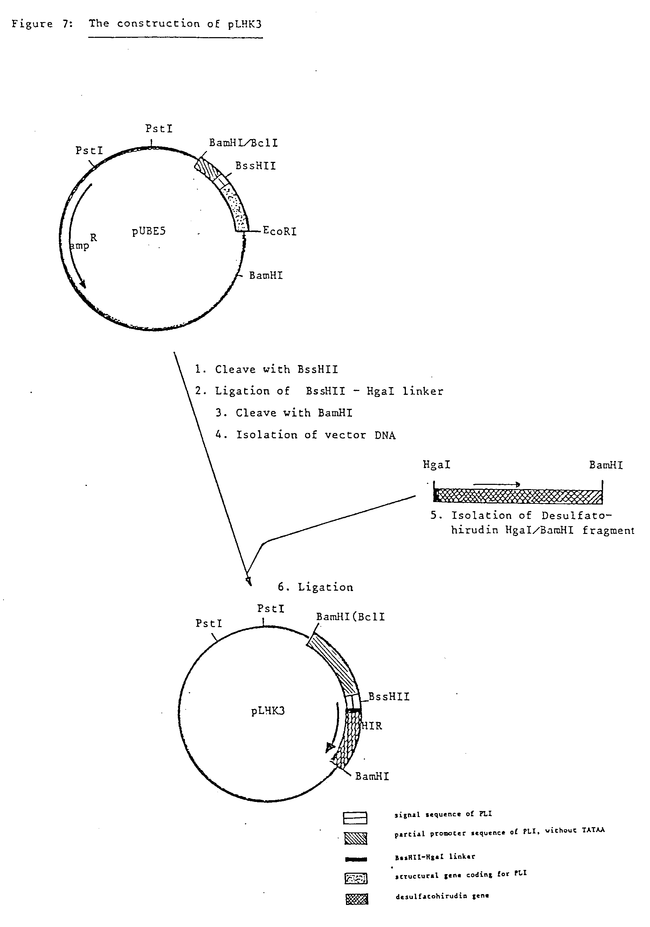

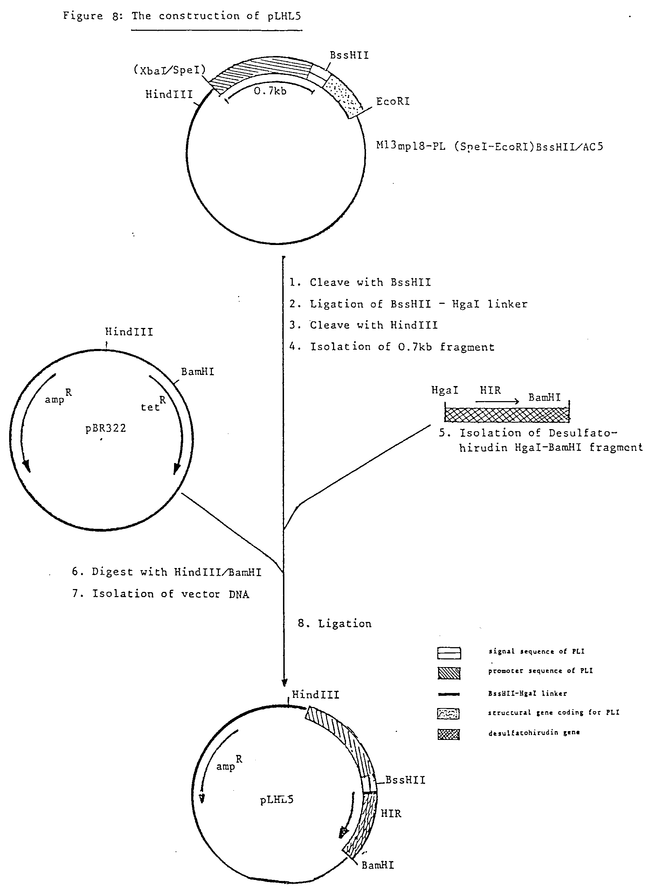

In a corresponding example the desulfatohirudin gene of pML31O (EP 168 342) is recloned into phage M13mp18-PL(SpeI-EcoRI)BssHII/AC5 (Fig. 6 to 9). A HgaI/BssHII linker is constructed, to make the HgaI restriction site of the desulfatohirudin gene of pML301L compatible to the BssHII site of M13mp18-PL(SpeI-EcoRI)BssHII/AC5. The Linker DNA is ligated to the cleaved phage DNA. The phage DNA carrying the linker fragment is cleaved with HindIII, which gives two fragments. The 0.7 kb fragment, comprising the signal structure of PLI and the linker fragment is isolated. A suitable replication plasmid, carrying a BamHI restriction site, such as pBR322, is cleaved with BamHI. The gene to be cloned is cleaved out of genomic DNA, plasmid or phage DNA with suitable restriction enzymes and, if necessary, is provided with the required compatible restriction sites.

Another method to make the restriction sites compatible is in vitro mutagenesis, as for example carried out by the construction of pML301L (Fig. 6). A linker containing an HgaI restriction site is constructed and attached to the 5'-terminal of the desulfatohirudin gene.

For higher expression rates, any eukaryotic terminator sequence can be ligated to the end of the structural gene. In a preferred embodiment of the invention, the terminator region of the PLI gene is used. For example an expression vector comprising the terminator site of PLI can be obtained by modification of the above described procedure in the following way. The promoter and secretional signal sequences are obtained from plasmid M13mp18-PL(SpeI-EcoRI)BssHII/AC5. The terminator region is obtained from pPL35-5. M13mp18-PL(SpeI-EcoRI)BssHII/AC5 is cleaved with BssHII and a BssHII-HgaI linker is ligated. In modification, plasmid pPL35-5 is cleaved with PstI, which gives two resulting fragments to which again a suitable linker, compatible with the 3' end of the cloned gene is ligated. The sticky ends of PstI restriction sites are filled up by the enzyme T4-Polymerase and a BamHI linker is added, for example available from Biolabs, New England. The pPL35-5 plasmid is cleaved with NheI and the resulting 0.7 kb fragment, comprising the terminator region of PLI, is isolated. For amplification any plasmid having compatible restriction sites can be used, e.g. pUC18. In the case of pUC18, the plasmid is cleaved with HindIII and XbaI. Four DNA fragments, the M13mp18-PL(SpeI-EcoRI)BssHII/AC5 fragment, carrying the Bss HII-HgaI linker, the 0.7 kB fragment of pPL35-5, carrying the BamHI linker, HindIII/XbaI cleaved pUC18 and the HgaI-BamHI fragment of the desulfatohirudin gene, are ligated to form pLHL7 (Fig. 9).

Bacteria are transformed by a conventional method and the transformants identified by their resistance, e.g. against tetracycline.

In particular the described expression vectors are amplified in suitable E. coli host strains, such as HB101, transformed (10) and selected by methods conventional in the art. The amplified plasmid DNA is isolated from the bacteria by conventional methods, in particular as described by Birnboim & Doly (17).

In a similar manner other plasmids with other homologous or heterologous genes can be constructed.

The DNA molecules of the present invention can also be prepared by an in vitro synthesis according to conventional methods. The in vitro synthesis is especially applicable for the preparation of smaller fragments of the PL expression system, e.g. of the DNA sequences coding for the promoter or especially the signal sequence of PLI, or mutants thereof.

DNA molecules of the invention comprising the PLI promoter and optionally the PLI signal sequence, the PLI structural gene, any other heterologous structural gene and/or the PLI terminator can also be used to transform filamentous fungi, such as Aspergillus, Penicillium or Cephalosporium, e.g. A. nidulans, A. oryzae, A. carbonarius and especially A. niger.

In order to allow selection of the transformed from the non-transformed fungi, the DNA molecules of the invention carry a selection marker or, alternatively, the fungi are cotransformed with a second vector containing such marker. As in other systems such selection marker is an expressible, structural gene, the expressed polypeptide of which (an enzyme) provides resistance against compounds toxic to the transformant or which completes the enzyme system of a mutant lacking such essential polypeptide. Such marker genes are far example the known qa-2, pyrG, pyr4, trpC, amdS or argB genes.

Within the frame of the invention a novel marker gene, named pyrA, was isolated from the genomic library of A. niger, which is related to and has similar function as pyrG of A. nidulans and pyr4 of N. crassa, namely producing the enzyme orotidine 5'-phosphate decarboxylase. This enzyme catalyses the decarboxylation of orotidine 5'-phosphate to uridylic acid (uridine 5'-phosphate) and also of fluoro-orotic acid to the toxic fluoro-uridine. An E. coli clone containing the pyrA gene was identified by hybridization with the 1.1 kb Hind III fragment of pDJB2 (26) containing part of the pyr4 gene, however, DNA of any other pyr gene coding for orotidine-5'-phosphate decarboxylase may be used. From a positive clone named E. coli B75183/pCG59D7, the plasmid pCG59D7, comprising the pyrA gene, was isolated and used for cotransformation of an A. niger pyrA⁻ mutant. Such pyrA⁻ mutant is defective in the orotidine 5'-phosphate decarboxylase gene and therefore is unable to produce the corresponding enzyme. Such mutant was prepared by treating conidiospores of A. niger N756 under mutating UV-irradiation and colonies surviving in the presence of fluoro-orotic acid and uridine are selected. Colonies surviving in the presence of fluoroorotic acid and absence of uridine are eliminated. The remaining uridine-requiring mutants, according to their ability of being transformable, belong to two complementation groups pyrA and pyrB, represented by mutants An8 and An10, respectively. They are treated in the farm of protoplasts thereof under transforming condition with the pyrA containing plasmid pCG59D7. Only the An8 colonies were found to be transformed and to contain the pyrA gene as evidenced by the hybridizing ability of digested DNA thereof with DNA of pUN 121.

The invention concerns also the selection marker plasmid pCG59D7, a host containing it and the pyrA mutant A. niger An8 and processes for their preparation.

The invention concerns further hosts transformed with the hybrid vectors of the invention and methods for their preparation. Such transformants are for example bacteria, such as E. coli, or filamentous fungi, such as Aspergillus, Penicillium or Cephalosporium, and in particular A. nidulans, A oryzae, A. carbonarius or preferably A. niger, e.g. A. niger An8. The invention concerns also a method for the preparation of such transformants comprising treatment of a host under transforming conditions with a recombinant DNA molecule, especially a hybrid vector, of the invention, optionally together with a selection marker gene and selecting the transformants.

The invention concerns also the use of the recombinant DNAs or a derivative thereof for the preparation of hybrid vectors which express useful polypeptides, e.g. PLI, desulfatohirudin and the like.

The invention concerns further a method for the preparation of polypeptides, characterized in that a hybrid vector of the invention is expressed in a suitable host and the polypeptide is isolated by conventional methods. This method comprises the overproduction of PLI in Aspergillus species by cultivating an Aspergillus host transformed with an expression hybrid vector for the expression and secretion of PLI and collecting the PLI from the nutrient medium.

### Short Description of the Figures

- Figure 1: shows the restriction map of plasmid pCG3B11 containing a Sau3AI fragment of the A. niger strain N756
- Figure 2: shows the restriction map of plasmid pPL29-5 containing the N-terminal EcoRI fragment of the DNA of formula I
- Figure 3: shows the restriction map of plasmid pPL35-5 containing the C-terminal EcoRI fragment of the DNA of formula I
- Figure 4: shows the DNA of the formula (I), with indications of some restriction sites, and the signal and structural amino acid sequences of PLI

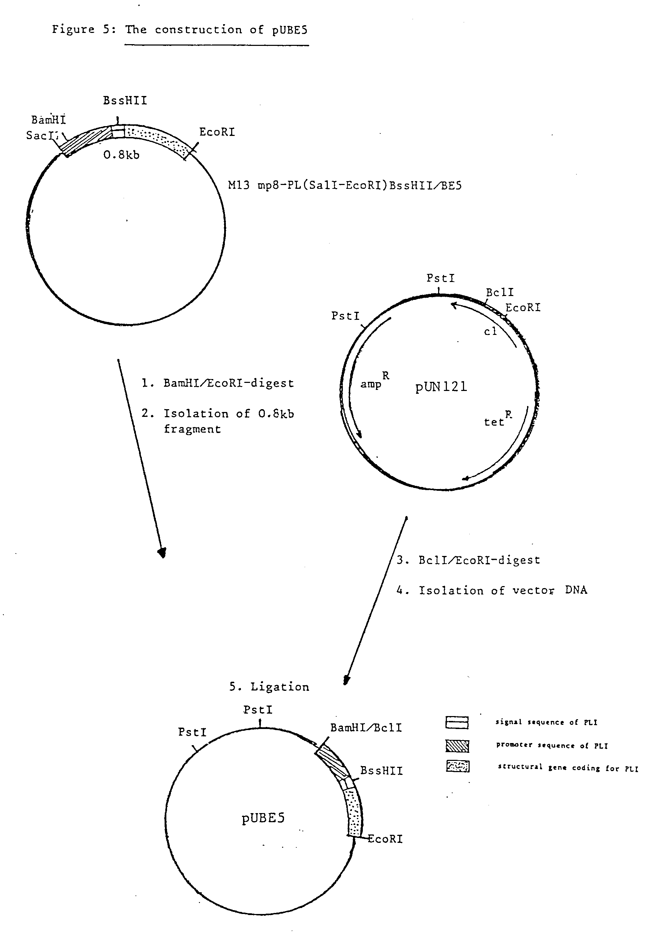

- Figure 5: shows the construction of pUBE5 from M13mp8-PL(SalI-EcoRI)BssHII/BE5 and pUN121. pUBE5 contains part of the PLI promoter, the signal sequence with BssHII restriction site, and part of the PLI structural gene
- Figure 6: shows the construction of pML310L containing the desulfatohirudin gene

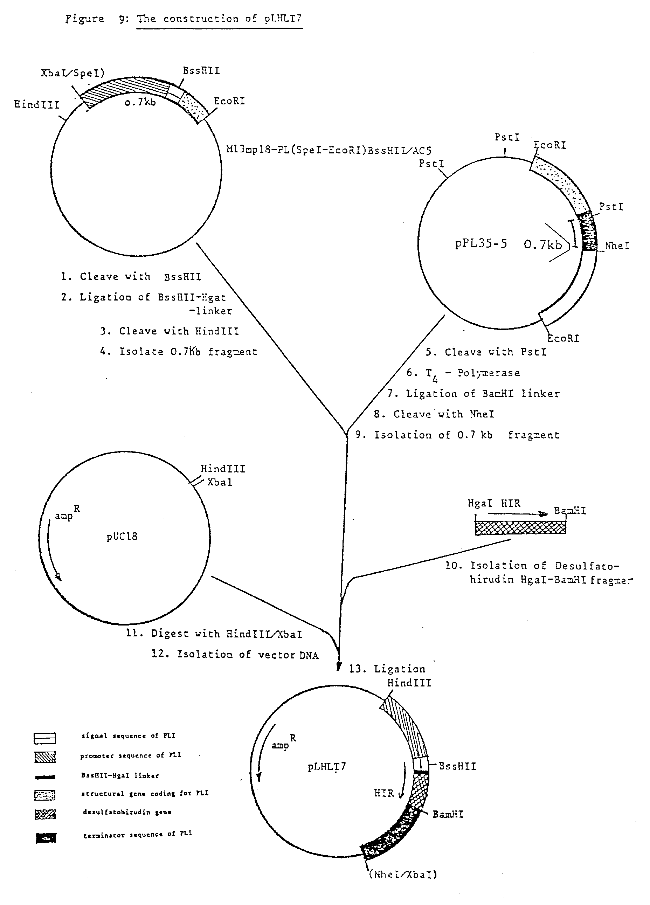

- Figure 7: shows the construction of pLHK3 containing part of the PLI promoter, the signal sequence with the BssHII restriction site, the linker BssHII-HgaI, and the desulfatohirudin gene
- Figure 8: shows the construction of pLHL5 containing the PLI promoter, the signal sequence with the BssHII restriction site and the desulfatohirudin gene
- Figure 9: shows the construction of pLHLT7 containing the PLI promoter, the signal sequence with the BssHII restriction site, the desulfatohirudin gene and the PLI terminator.

The following examples serve to illustrate the invention, however are in no way intended to restrict it.

### The abbreviations have the following meanings:

- bp: base pairs
- BSA: Bovine serum albumin
- cpm: counts per minute (radioactive decay)
- dATP: 2'-deoxyadenosine triphosphate
- dCTP: 2'-deoxycytidine triphosphate
- dGTP: 2'-deoxyguanosine triphosphate
- dTTP: 2'-deoxythymidine triphosphate
- dNTP: mixture of dATP, dCTP, dGTP and dTTP
- CIAP: alkaline phosphatase from calf intestine
- DNA: deoxyribonucleic acid
- DTT: 1,4-dithiothreitol
- EDTA: ethylenediaminetetraacetic acid disodium salt
- hrs: hours
- IPTG: isopropyl-β-D-thio-galactopyranoside
- kb: kilobases
- min: minutes
- PL: pectin lyase I
- PTH: phenylthiohydantion
- RF-DNA: double-stranded replicative form DNA
- RNA: ribonucleic acid
- rpm: revolutions per minute
- SDS: sodium dodecyl sulfate
- ss: single-stranded
- RT: room temperature
- Tris: tris(hydroxymethyl)-aminomethane
- tRNA: transfer RNA
- U: units
- µg: microgram
- vol: volume
- X-GAL: 5-bromo-4-chloro-3-indonyl-β-galactoside

### Buffers, media, reagents:

- ss-Denhardt: 0.02 % ficoll (Sigma), 0.02 % polyvinylpyrrolidone (Sigma), 0.02 % BSA (Sigma), 100 µg/ml denaturated salmon sperm DNA (Sigma)
- EcoRI buffer: 0.01 M Tris HCl pH 7.5 0.1M NaCl 0.01 M MgCl₂. 1 mM 2-mercaptoethanol
- HgaI buffer: 50 mM NaCl, 10 mM MgCl₂, 1 mM DTT, 100 µg/ml BSA, 6 mM Tris-HCl pH 7.4
- IPTG: 100 mM Isopropyl-β-D-thio-galactopyrano side (23.8 mg/ml in H₂O) prepare just before use
- LB medium: 1 % Bacto-tryptone (Difco), 0.5 % Bacto yeast extract (Difco), 170 mM NaCl, adjusted to pH 7.5 with NaOH
- ligation buffer: 20 mM Tris-HCl, 10 mM MgCl₂, 10 mM dithioerythritol, 0.6 mM ATP, pH 7.6
- TBE-buffer: 1 litre contains 10.8 g Tris, 5,5 g boric acid, 4 ml 0.5 M EDTA (pH 8.0)
- TE buffer: 10 mM Tris-HCl (pH 7.5), 1 mM EDTA
- low TE buffer: 10 mM Tris-HCl (pH 7.5), 0.1 mM EDTA
- top agar: per litre 10 g bacto tryptone, 8 g NaCl, 8 g agar
- 2xTY medium: per litre 16 g bacto tryptone, 10 g yeast extract, 5 g NaCl
- X-GAL: 2 % (5-bromo-4-chloro-3-indonyl-β-galactoside) in dimethylformamide prepare just before use
- SOC medium: 2 % Bacto Tryptone (Gibco), 0.5 % Yeast-Extract (Gibco), 10 mM NaCl, 2.5 mM KCl, 10 mM MgCl₂, 5 mM MgSO₄, 20 mM Glucose
- kinase buffer: 50 mM Tris-HCL, 10 mM MgCl₂ 5 mM DDT (pH 7.5)
- X-gal plates: 0.002 % X-Gal, 0.07 mM IPTG
- NET: 0.15 M NaCl, 0.015 M Tris·HCl (pH 7.5) 1mM EDTA
- minimal medium for E. coli: 0.6 % Na₂HPO₄, 0.1 % NH₄Cl, 0.05 % NaCl, 1 mM MgSO₄, 0.01 mM CaCl₂, 1mM thiamine-HCl, 0.2 % glucose
- minimal medium for A. niger: 1 liter contains 6 g NaNO₃, 0.52 g KCl, 0.52 g MgSO₄ x 7 H₂O, 1.52 g KH₂PO₄, traces of Zn, Fe, 10 g glucose, adjusted to pH 6.5 with NaOH
- complete medium for A. niger: Mycophil-Agar (BBL)
- PCT: 25 % polyethylene glycol 6000 (Merck, Darmstadt) in 10 mM Tris·HCl (pH 7.5), 50 mM CaCl₂
- sporulation medium: 10 g/l phytone peptone, 10 g/l glucose, 10 g/l agar
- SSC: 0.15 M NaCl, 0.015 M sodium citrate

For plates all media are solidified by addition of 1.5 % Bacto agar (Difco).

### The following strains are used:

E. coli strain HB101: F⁻,hsdS20(r⁻_{B}m-B), recA13, ara14, proA2, lacY1, galK2, rpsL20(str^{R}), xyl-5, mtl-l, supE44, λ⁻ (Maniatis et al. (8))
E. coli strain BJ5183: F⁻, endA, sbcB⁻, recBC⁻, galK, met⁻, str^{R}, thi-1, bioT, hsdR(r⁻_{K}, m_{K}⁺), λ⁻ (Hanahan (10)).
E. coli strain JM101: supE, thi, Δ(lac-proAB), (F',traD36, proAB,lac19ZΔM15) (Yanish-Perron et al. (25))
Aspergillus niger strain N756: The A. niger strain N756 has been selected for high production of pectinolytic complex enzymes.

### The following vectors are used:

### M13mp phage

The M13mp8,9,18,19 vectors (28) are derivatives of the single-stranded DNA bacteriophage M13 and are designated to facilitate DNA sequencing by allowing cloning of DNA fragments at a versatile polylinker site and the cloning of the same restriction fragment in both possible orientations. Sequences cloned into these vectors can readily be used as templates for sequencing reactions or the production of single-stranded probes using a standard oligodeoxy-ribonucleotide primer and the Klenow fragment of the E. coli DNA polymerase I. The vector DNA is carrying the E. coli lac-operon promoter and the genetic information of the first 145 amino acids of β-galactosidase. The polylinker sequences containing multiple restriction sites are inserted into the lacZ sequence. The polylinker retains the lacZ reading frame and the vector gives allelic complementation of a LacZα host strain, yielding blue plaques on plates containing IPTG and X-gal. Recombinant phages containing inserts that destray the reading frame or otherwise interfere with expression of the lacZα peptide are revealed as colorless plaques.

### pUC18 plasmid

The pUC18 plasmid (28) can be used for cloning DNA sequences in the lacZα region providing a detection for transformants containing recombinant plasmids on the basis of their Lac phenotype. Bacteria harboring plasmids without inserts make blue colonies on indicator plates (plates with X-gal) whilst those harboring recombinant plasmids make white colonies. Plasmid pUC18 contains unique cloning sites in the lacZα region fitting to those of M13mp18 phage. The plasmid carries a gene for amp^{R}.

### pML 310 plasmid

pML 310 is described in the European Patent Application No. 0168342, pML310 is characterized through amp^{R} , the trp promoter and a gene coding for desulfatohirudin, a thrombin inhibitor which naturally occurs in leeches.

### pUN121 plasmid

Plasmid pUN121 [(Nilsson et al. (9)] can be used as a cloning vector that allows positive selection of transformants harboring plasmids with DNA inserts. The plasmid contains the cI gene of bacteriophage lambda, and the tet gene. Bacteria harboring pUN121 are sensitive for tetracycline since the tet gene is transcribed from the right promoter of bacteriophage Lambda and this promoter can be repressed by the cI-encoded repressor. Insertion of DNA fragments in one of the sites in the cI gene inactivates the repressor gene, yielding tetracycline-resistant transformants. In addition plasmid pUN121 has a functional amp gene so that amplification of plasmid DNA can be performed under selective conditions.

### pBR322 plasmid

Plasmid pBR322 carries genes for resistance to the antibiotics ampicillin (amp^{R}) and tetracycline (tet^{R}). The plasmid carries several unique cloning sites, of which some are located in either the amp or tet gene so that insertion of cloned DNA leads to antibiotic-sensitive bacteria.

### pDJB2 plasmid

This plasmid has been described by Ballance and Turner (26)

### Example 1: Isolation and characterisation of pectine lyase I

### Example 1.1: Amino acid sequence determination of the N-terminal part of pectin lyase I

Pectin lyase I is purified from Ultrazym® (a mixture of pectinolytic enzymes obtained from A. niger, Novo Industries, Copenhagen) in analogy to the method described by F.E.A. van Houdenhoven (1). Two mg of pectin lyase I are dialyzed against several liters of distilled water and subsequently lyophilized. The protein is dissolved in 1.5 ml 100 mM NaHCO₃, pH 10.0 and kept at room temperature for 3 hrs. This treatment turns out to increase the number of steps, which can be reliably sequenced by the automatic Edman degradation method (2). After the alkaline treatment the protein is dialyzed against 1 Liter of distilled water, 1 % formic acid and distilled water subsequently. Automatic Edman degradation is performed in a Beckman spinning-cup sequenator, model 890 C. The intact reduced and carboxymethylated protein is degraded using a quadrol single-cleavage program (modification of Beckman program 072172 C). The degradation of the peptide is carried out using a dimethylbenzylamine single-cleavage program (Beckman program 082773) or the program of Hunkapiller and Hood. To prevent wash-out of the peptide, 2.5 mg of cod parvalbumin is used as a carrier (3).

Phenylthiohydantoin derivatives of amino acids are identified by high-performance liquid chromatography according to the method of Frank and Strubert (4). The following N-terminal amino acid sequence is determined for pectin lyase I:
V-G-V-X₄-G-T-P-E-G-F-A
Amino acid X₄ is very likely to be ser but its presence has not been established unambiguously.

### Example 1.2: Preparation of cyanogen bromide fragments of pectin lyase I

Reduction and S-carboxymethylation of pectin lyase I is performed according to Crestfield et al. (5). 3 g urea (ultra pure) are dissolved in 5 ml of distilled water and stirred with 0.5 g of a mixed-bed ion-exchanger (Biorad Ag 50 1-X8) for 1 h. After removal of the ion-exchange beads, 10 mg EDTA and 200 mg Tris are added to 4 ml of the urea solution and the pH is adjusted to 8.5 with HCl. The solution is filled up to a final volume of 5 ml in a vial with distilled water. Then 5-20 mg of pectin lyase I purified as described by F.E.A. van Houdenhoven (1) are added and the vial is maintained under a nitrogen barrier.

Finally 33 µl of β-mercaptoethanol are added and the reduction is continued for 4 hrs at room temperature under a nitrogen barrier. 0.33 ml of a freshly prepared solution (0.268 g iodoacetic acid in 1 ml 1N NaOH) are added under nitrogen to the reaction mixture and the mixture is kept in the dark for 15 min at room temperature.

The reaction is stopped by addition of β-mercaptoethanol. Subsequently the carboxymethylated protein is applied to a Sephadex G-25 gel filtration column (100 ml bed volume) which is wrapped in aluminum foil and eluted with 0.5 % formic acid. The protein fractions are pooled and lyophilized.

The carboxymethylated pectin lyase I (2-10 mg) is dissolved in 70 % formic acid and solid cyanogen bromide is added in approx. hundred fold molar excess to methionine of which 1 residue occurs per pectin lyase I. The reaction mixture is stirred continuously for 24 hrs at room temperature in a closed reaction vial. Samples of 5 µl are taken at regular time intervals and analyzed an 15 % SDS-PAGE to check the extent to which the protein is split. After 24 hrs water is added and the preparation is partially evaporated by flushing with nitrogen. This step is repeated to remove the formic acid.

The SDS-PAGE analysis identifies, besides some residual pectin lyase I, two cyanogen bromide peptides, CBI and CBII with apparent molecular weight of 16.5 kD and 30 kD. Purification of these fragments is performed by gel permeation chromatography using a Sephacryl-S200 column (length 190 cm, ⌀ 1 cm) equilibrated in 20 mM sodium phosphate buffer pH 6.0, 100 mM NaCl, 4 M urea and 1 % (v/v) β-mercaptoethanol. The CB II fragment appears in two peaks, one peak eluting even before the residual intact protein which therefore must represent an aggregate and another peak in between the intact protein and the small CBI fragment.

### Example 1.3: Amino acid sequence determination of the N-terminal part of CB II of pectin lyase I

200 µg of the purified non-aggregated CB II fragment of Example 1.2. are used for the automatic Edman degradations (2) using a Beckman spinning-cup sequenator. Conversion to the PTH amino acids is realized after evaporation of the fractions and the addition of 200 µl 2N HCl in methanol to the residue for 15 min at 65°C. The solution is then evaporated again and the residue is taken up in 50 µl THF/CH₃CN (1:1). The PTH-amino acids are identified by HPLC using a Zorbax CN^{R} column (Du Pont) according to R. Knecht et al. (6).

The N-terminal amino acid sequence of CB II is the following:
V-S-G-V-S-N-I-I-I-Q-N-I-A-V-X₁₅-D-I-N-X₁₉-E
X₁₅ and X₁₉ are amino acid residues which have not been identified.

### Example 2: Construction of a genomic library of Aspergillus niger

### Example 2.1: Isolation of high molecular weight DNA from Aspergillus niger

The isolation of high molecular weight A. niger DNA is done according to Yelton et al. (7). Conidiospores of A. niger strain N 756 are inoculated in 200 ml minimal medium containing 0.1 % arginine and shaken in 500 ml flasks with one well at 28°C on a rotary shaker for 2-3 days. The mycelium is harvested by filtration, washed with cold sterile water, frozen and powdered in liquid nitrogen. The cells are rapidly suspended in 20 ml 50 mM EDTA pH 8.5, 0.2 % SDS and 20 µl diethylpyrocarbonate and lysated by vigorously shaking for 1 min at room temperature. The lysate is heated to 68°C for 15 min, cooled to room temperature and centrifuged for 15 min at 12000 g at RT. 16 ml of the supernatant are transferred to a new tube and after addition of 1 ml 8 M potassium acetate pH 4.2 left on ice for 1 hr. The precipitate is centrifuged at 25000 g for 15 min at 4 C°. 12 ml of the supernatant are recovered and the nucleic acids precipitated with 12 ml of isopropanol. The precipitate is pelleted by centrifugation for 1.5 min at 12000 g and the pellet resuspended in 2 ml TE containing 10 µg/ml RNAseA (Boehringer, Mannheim). The DNA fragments are extracted with chloroform/phenol and precipitated with ethanol as described by Maniatis et al. (8) at pages 458-459 and 461-462.

### Example 2.2: Cloning of high molecular weight DNA fragments from Aspergillus niger into pUN121

The high molecular weight DNA of Example 2.1. (100 µg) is precipitated by centrifugation and the pellet is resuspended in 750 µl buffer consisting of Tris-HCl 6 mmol/l, NaCl 50 mmol/l, MgCl₂ 6 mmol/l, pH 7.5. Partial digestions are carried out with Sau3AI, the concentrations being in the range of 0.25-0.01 U/µg for 60 min at 37°C. The reactions are terminated by addition of EDTA to final concentrations of 20 mM.

The partially digested DNA fragments are separated on a 0.45 % agarose gel. Lambda DNA digested with HindIII is used as marker to determine the size of the partially digested DNA fragments. The gel region containing the desired fragments in the range of 15 kb to 20 kb is cut out and transferred to the sample well of an ISCO electrophoretic concentrator (ISCO GmbH), covered with 0.1 TBE and electroeluted at 1 Watt for 90 min. The DNA fragments are extracted with chloroform/phenol and precipitated with ethanol.

4 µg of the partially digested DNA fragments are resuspended in H₂O and ligated for 15 hrs at 15°C to 1 µg of pUN121 plasmid DNA (Nilsson et al. (9)), linearized with BclI, in a total volume of 106 µl ligation buffer with 6U T4 DNA ligase (Boehringer, Mannheim). 10 µl aliquots of this annealing mixture are added to 210 µl of competent E. coli BJ5183 cells, which have been prepared for transformation as described by Hanahan (10). The cells are kept on ice for 30 minutes and heated to 42°C for 90 sec, replaced on ice for 2 min, 800 µl SOC-medium are added and the cells are incubated at 37°C for 1 hr. The cells are spread on 10 cm agar plate containing LB-medium supplemented with 8 mg/l tetracyciin (SIGMA). The plates are incubated at 37°C for 16 hrs. About 6000 transformed colonies are obtained characterized by their tetracyclin resistance. The efficiency is about 12000 to 20000 tetracycline-resistant colonies per µg of pUN121 DNA.

The plasmid analysis of 11 randomly chosen recombinant clones indicates an average DNA insert of 10 kb. To represent the genome of A. niger (4.5 x 10' bp) in the library almost 4 times, 15360 tetracyclin resistant colonies are picked and grown individually over night in the wells of microtiter dishes in LB-medium supplemented with 8 mg/l tetracycline. After incubation glycerol is added to 50 % v/v and the microtiter dishes are stored at -70°C.

### Example 3: Screening the genomic library of A. niger for nucleic acids related to PLI

### Example 3.1: Preparation of filter replicas of the library

For the isolation of the pectin lyase I gene from the DNA library obtained as described in Example 2.2., filter replicas from the 15360 chosen transformed E. coli BJ5183 cells are made following the method described by Gergen et al. (11). The cells are transferred from the microtiter dishes to agar plates supplemented with 8 mg/l tetracycline by using a stamp and grown over night at 37°C. Conveniently cut Whatman 541 filter papers (114 x 175 mm per 192 clones) are placed on top of the colonies. After 2 hrs at 37°C the filters are transferred to LB plates containing 250 mg/ml chloramphenicol (Sigma, St. Louis, USA) and incubated over night at 37°C. The filters are washed twice in 0.5 M NaOH, twice in 0.5 M Tris HCl, pH 7.4, washed twice in SSC and are air dried. The filter replicas can be used for several hybridisation experiments, each preceeded by the above mentioned washing steps.

### Example 3.2: Synthesis of oligonucleotide mixtures

The oligonucleotides for screening the DNA library of A. niger, obtained as described in Example 2.2., are synthesized corresponding to the amino acid sequence determined in Examples 1.1. and 1.3. by using the phosphoramidite method (M.H. Caruthers (12), with an Applied Biosystem (Model 380B) oligonucleotide synthesizer.

### 3.2.1: Synthesis of an oligonucleotide coding for the N-terminus part of pectin lyase I of Aspergillus niger

The oligonucleotide coding for the N-terminus of the pectin lyase I protein is synthesized with respect to its aminoacid composition as determined in Example 1.1. Because of genetic degeneration 256 oligonucleotides are required to comprise all possibilities.

Two separate mixtures of oligonucleotides are chemically synthesized, because for technical reason it is necessary to reduce the number of nucleotides in the mixture. Both contain 128 different oligonucleotides, each being a complementary strand of the strands coding for the amino acid sequence T₆-P-E-G-F-A₁₁. In the following they are named mixture 2014 and 2015, and are composed of oligonucleotides as follows:
mixture 2014 3'TGR₁ GGR₁ CTR₂ CCR₃ AAR₃ CG5'
mixture 2015 3'TGR₁ GGR₁ CTR₂ CCR₂ AAR₃ CG5'
wherein R₁ is A, C, G or T, R₂ is C or T and R₃ is A or G.

### Example 3.2.2: Synthesis of an oligonucleotide coding for the N-terminus part of the C-terminal fragment of the CNBr decomposed pectin lyase I protein

The oligonucleotide coding for the N-terminus part of the C-terminal fragment of the CNBr decomposed pectin lyase I protein is synthesized with respect to its amino acid composition as determined in Example 1.3. A mixture of 108 oligonucleotides is synthesized being a complementary strand of the strands coding for the amino acid sequence N₁₃₄-I-I-I-Q₁₃₈, and are composed of oligonucleotides as follows.
mixture 2060 5'AAR₁ ATR₂ ATR₂ ATR₂ CAR₃ A 3'
wherein R₁ is C or T, R₂ is A, T or C and R₃ is A or G.

### Example 3.2.3: ³²P-labelling of the oligonucleotide mixtures

67 pmoles of each oligonucleotide mixture 2014, 2015 and 2060 of Example 3.2.1 and 3.2.2 are 5'labelled using 50 pmoles of [γ³²P]ATP (Amersham, Buckinghamshire, England, 5000 Ci/mmol). The incubation is carried out at 37°C with 150 units T4 polynucleotide kinase (New England, Nuclear) in 500 µl kinase buffer. The ligation reaction of radioactive labelled ATP to the oligonucleotides is detected by running an aliquot of the reaction mixtures on a 20 % polyacrylamide gel followed by autoradiography.

### Example 3.3: Screening of the gene library with radioactive labelled oligonucleotide mixtures 2014 and 2015

The screening of the gene library is carried out by hybridisation with the radioactively labelled oligonucleotide mixtures 2014 and 2015 of Example 3.2.1 Batches of 20 filter replicas of the gene library (Example 3.1.) are wetted in 6 x NET and are prehybridized in 200 ml 6 x NET, 1xss-Denhardt, 0.1 % SDS at 49.3°C for 4 hrs. Then 67 pmoles of the radioactively labelled oligonucleotide mixture 2014 are added and the hybridisations carried out over night at 49.3°C. The same procedure is carried out using radioactively labelled oligonucleotide mixture 2015. The filters are washed two times for 5 minutes in 2 x SSC, 0.1 % SDS at room temperature, two times for 1 hr in 2 x SSC, 0,1 % SDS at 49.3°C and one time for two hrs in 0.2 x SSC, 0.5 % SDS at 49.3°C. The filters are air dried and autoradiographed for 3 days using KODAK X-omat SO282 films.

With the oligonucleotide mixture 2014 33 clones and with the oligonucleotide mixture 2015 39 clones are found, which give a rather strong hybridisation response. To establish a sublibrary the 72 clones are grown up in a new microtiter dish and transferred to Whatman 541 filter as described in Example 3.1. Two filter replicas of the sublibrary are hybridized with both probes individually, washed and autoradiographed. With the oligonucleotide 2014 mixture only the clones obtained by hybridisation with the oligonucleotide 2014 mixture hybridize. Likewise with the oligonucleotide 2015 mixture only the clones obtained by hybridisation with the oligonucleotide mixture 2015 hybridize.

### Example 3.4: Screening the sublibrary with the oligonucleotide mixture 2060

The filter replicas of the sublibrary (72 clones), obtained in Example 3.3, are wetted in 6 x NET and prehybridized for 4 hrs in 15 ml 6 x NET, 1 x ss-Denhardt, 0.1 % SDS at 32°C. 8 pmoles of oligonucleotide mixture 2060, radioactive labelled as described in Example 3.2.3, are added and the hybridization carried out over night at 32°C. The filters are washed two times for 5 minutes each in 2 x SSC, 0.1 % SDS at room temperature, two times for 1 hour each in 2 x SSC, 0.1 % SDS at 32°C and one time for two hours in 0.2 x SSC, 0.5 % SDS at 32°C. The filters are air dried and autoradiographed for 3 days using KODAK X-omat SO282 films. 3 positive clones are found, all belonging to the group that hybridize with the 2014-probe as described in Example 9; one clone is named E. coli BJ5183/pCG3B11 (short 3B11).

### Example 4: Isolation of plasmid pCG3B11 and restriction analysis thereof

Large plasmid preparations (14) are made of the clone 3B11, obtained as described in Example 3.4. The corresponding plasmid, named pCG3B11, is analysed by complete restriction digestion with HindIII, EcoRI and PstI (all Boehringer, Mannheim) and electrophoretic separation over a 1 % agarose gel (Figure 1).

### Example 5: Subcloning of the EcoRI fragments of pCG3B11 containing the N-terminal and the C-terminal fractions of the pectin lyase I gene

### Example 5.1: Isolation of Sau3AI fragments of pG C3B11 and ligation into M13DNA

1 µg of plasmid pGC3B11 (Example 4) is digested to completion with 18 units of restriction endonuclease Sau3AI (Boehringer, Mannheim) in 20 µl 10 mmol/l Tris-HCl, 75 mmol/l NaCl, 10 mmol/l MgCl₂, pH 7.2 for 1 hour at 37°C. The reaction is terminated by addition of EDTA to a final concentration of 20 mM. The DNA fragments are extracted with phenol/chloroform precipitated by ethanol and resolved in 20 µl TE buffer. 100 ng of the Sau3AI digested DNA is ligated to 20 ng of M13mp8 phage DNA (15) linearized by BamHI digestion (Boehringer, Mannheim). The ligation is carried out for 4 hrs, at 15°C with 1 unit of T4 DNA ligase (Boehringer, Mannheim) in a total volume of 10 µl ligation buffer. Competent E coli JM101 cells are made in 50 mM CaCl₂ in accordance to the Amersham handbook (16) p. 25. 0.3 ml aliquots of the competent JM101 cells are transferred to 15 ml sterile culture tubes on ice. 5 µl of ligated phage DNA are added to each tube. The mixtures are kept on ice for 40 min. The cells are heat shocked to 42°C for 3 min and the tubes returned to the ice bath. For each tube the following mixtures are prepared: 40 µl IPTG 100 mM, 40 µl X-gal 2 % in dimethylformamide and 200 µl E. coli JM101 cells from a fresh exponentially growing culture. 270 µl of this mixture are added to each tube containing heat shocked E. coli JM101 cells. 3 ml of molten top agar (kept at 42°C) are added to each tube and the tubes are poured onto agar plates. The plates are incubated inverted at 37°C overnight.

### Example 5.2: Screening of recombinant phages with the oligonucleotide probe 2060

E. coli JM101 transformed with the recombinant phage DNA show white plaques on the X-gal containing agar plates of Example 5.1., 384 of which are picked and grown individually at 37°C in 1.5 ml 2 x TY medium inoculated with 15 µl exponentially growing E. coli JM101 cells. After 6 hrs the 384 cultures are centrifuged for 5 min in an Eppendorf centrifuge and the phage supernatants stored at 4°C. 100 µl from each of the 384 supernatants are transferred to four Gene Screen membranes (New England Nuclear) by using a BioDot apparatus with 96 slots (BioRad). The membranes are soaked 5 min in 0.5 M NaOH, 1.5 M NaCl and 5 min in 3 M NaCl, 1 M Tris-HCl, pH 5.6, dried in air and baked in vacuo at 80°C for 2 hrs. Finally the four membranes are prehybridized, hybridized with the oligonucleotide mixture 2060 radioactively labelled as described in Example 8, washed and autoradiographed as described in Example 3.4. From the ligation experiment of Example 5.1. one positive, recombinant phage is chosen for further analysis, designated as mp8-3A.

### Example 5.3: Sequence analysis of recombinant mp8-3A phage

The supernatant containing phage mp8-3A obtained as described in Example 5.2., is used to prepare single-stranded and replicative form DNA (RF-DNA) as described in the Amersham handbook (16) at p. 18-27. The single-stranded templates are sequenced using the chain terminator method described in the Amersham handbook at p. 30-35. The phage proves to contain the 574 bp Sau3AI fragment with the predicted nucleotide sequence of oligonucleotide probe 2060 at position 584 to 599.

### Example 5.4: Identification of the N-terminal and the C-terminal EcoRI fragments of pectin lyase I gene

The sequenced EcoRI restriction site of phage mp8-3A (Example 5.3.), is used to identify and subclone two EcoRI fragments, one containing the C-terminal part of PLI gene, comprising the terminator region, and one containing the N-terminal part of the gene comprising the promotor region. The following two oligonucleotides are synthesized as described in Example 3.2. Oligonucleotide 5052: 5'TGGATGATGATGTTGGAGAC3'
starts at position 597, 5' to the central EcoRI-site at position 649/650, and extends to position 577 (complementary strand). Oligonucleotide 5066: 5'AGGTCCAGGACAACACCTAC3'
starts at position 1020, 3' to the central EcoRI-site, and extends to position 1039.
The oligonucleotide mixtures 5052 and 5066 are radioactive labelled as described in Example 3.3.

1 µg of plasmid pCG3B11 (Example 11) DNA is digested to completion with EcoRI (Boehringer, Mannheim) and the fragments separated on a 1 % agarose gel. The DNA fragments are blotted to Gene Screen membrane (New England Nuclear) as described by the supplier p. 383-386 and the membranes hybridized with radioactively labelled probes of oligonucleotides 5052 and 5066 as described by Maniatis et al. (8) p. 387-389. Probe 5066 hybridizes with a 3.5 kb EcoRI fragment which contains the C-terminal part and the terminator region of the PLI gene. Probe 5052 hybridizes with a 2.9 kb EcoRI fragment containing the N-terminus and the promoter region of the gene.

### Example 5.5: Construction of pPL29-5 and pPL35-5 by recloning of EcoRI fragments of pCG3B11 into pUN121

4.5 µg of plasmid pCG3B11 DNA obtained as described in Example 4 are incubated with 50 units EcoRI (Boehringer, Mannheim) in 50 µl 0.01 M Tris-HCl pH 7.5, 0.1 M NaCl, 0.01 M MgCl₂, 1 mM mercaptoethanol for 2 hrs at 37°C. The DNA fragments are separated on a 1 % agarose gel, the gel slice containing the 2.9 kb fragment and the 3.5 kb fragment are eluted as described in Example 5 and the DNA extracted with phenol/chloroform and precipitated by ethanol. The precipitates are resuspended in 40 µl low TE buffer and 10 µl of this solution are ligated to 100 ng pUN121 (9) linearized by EcoRI digestion. The ligation is carried out at 15°C for 4 hrs with 5 units T4 DNA ligase (Boehringer, Mannheim) in 35.5 µl 20 mmol Tris-HCl, 1 mmol EDTA, dithioerythritol 5 mmol, 60 mmol KCl, 50 % glycerol, pH 7.6.

17.5 µl aliquots of this annealing mixture are added separately to 200 µl of competent E. coli HB101 cells, which have been prepared for transformation by treatment with calcium chloride as described by Maniatis et al. (8), p. 250. The mixtures are kept on ice for 30 min and heated to 42°C for 2 min, then diluted with 1 ml of SOC-medium and incubated at 37°C for 1 h. The cells are collected by centrifugation at 2000 g for 5 min. Each cell pellet is resuspended in 600 µl of SOC-medium and spread on three agar plate containing LB-medium supplemented with 8 mg/l tetracyclin. The plates are incubated at 37°C for 16 h.

The plasmids of 6 recombinant tet^{R} clones are isolated from each transformation by the method of Birnboim & Doly (17) and analysed by restriction analysis. One clone containing the 2.9 kb EcoRI fragment of pCG3B11 is chosen for further analysis and named pPL29-5 (Fig 3). Another clone containing the 3.5 kB EcoRI fragment of pCG3B11 is chosen and named pPL35-5 (Fig. 3).

### Example 6: Sequence determination of the pectin lyase I gene

The DNA of pPL29-5 (Example 5.5.) containing the N-terminal part of the PLI gene and the DNA of pPL35-5 (Example 5.5.) containing the C-terminal part of the PLI gene are isolated as described by Humphreys et al. (14). Parts of the inserts of plasmids pPL29-5 and pPL35-5 are obtained by restriction with suitable enzymes. The fragments are subcloned into M13mp8 and M13mp18 and sequenced using the M13 Cloning and Sequencing Kit (M15 Sequencing Kit N.4502, Amersham) under the conditions given by the supplier (16).

The entire sequence of the 2.7 kb SpeI-NheI fragment, comprising the complete sequence of PLI gene, is shown in Figure 4. It comprises 688 nucleotides of the promoter region, 1369 residues of the structural part of the PLI gene and 660 nucleotides of the terminator region.

The sequence of PLI, corresponding to amino acid sequence of the N-terminus of PLI as determined by amino acid sequence analysis (Example 1), is preceded by 57 nucleotides coding for the signal sequence:
M₁-K-Y-A-A-A-L-T-A-I-A-A-L-A-A-R-A-A-A₅₇
A computer aided search for consensus sequences of exon/intron splice junctions as well as for intron internal sequences (Boel E. et al. (18) and Mount S.M. (19)) leads up to postulate the presence of four introns with length of 65 bp, 62 bp, 63 bp and 57 bp respectively (Fig. 4, underlined).

The sequence of the PLI gene is determined by combination of the sequences of the EcoRI-SpeI fragment of plasmid pPL29-5 and the EcoRI-NheI fragment of pPL35-5 (both determined by subcloning in M13).

### Example 7: Construction of expression vectors

### Example 7.1: Introduction of a new restriction site in the signal sequence of the PLI gene

The introduction of a new BssHII site in the signal sequence region of the PLI gene (sequenced in Example 6) by in vitro mutagenesis is done according to Zoller and Smith (20). An oligonucleotide consisting of 19 residues is synthesized by the method described in Example 3.2. which is complementary to the DNA sequence (pos. 36-54) coding for the C-terminal part of the PLI signal sequence, with the exception of nucleotide 10 (45) leading to a C/G transversion (mutagenic primer). The exchanged nucleotide 45 of the mutagenic primer is marked by *. 
part of PLI signal sequence   5'CCTCGCTGCCCGCGCCGCT3'
                                 •   •         •   •
                                 36  40        50  54
                                 •   •    *    •   •
mutagenic primer 5156:           5'CCTCGCTGCGCGCGCCGCT3'
For the in vitro mutagenesis 200 pmols of oligonucleotide 5156 is 5' phosphorylated with 20 nmols rATP. The reaction is carried out for 1 h at 37°C with 10 units T4 Polynucleotide kinase (Boehringer, Mannheim) in 20 µl kinase buffer. The reaction is terminated by heating to 65°C for 10 min.

### Example 7.2: In vitro mutagenesis of template M13mp8-PL (Sal-EcoRI) DNA

The first in vitro mutagenesis experiment concerns single stranded DNA from phage M13mp8-PL (SalI-EcoRI), obtained in Example 6, which contains the 0.8 kB SalI-EcoRI fragment of plasmid pPL29-5, containing 117 nucleotides of the promotor and the N-terminal part of the PLI gene. 1 pmol single-stranded M13mp8-PL(SalI-EcoRI) DNA is mixed with 20 pmoles mutagenic primer 5156 (Example 7.1.) and 10 pmoles universal M13 sequencing primer (N.4511, Amersham) in 10.5 µl 0.02 M Tris-HCl pH 7.5, 0.01 M MgCl₂, 0.05 M NaCl, 0.001 M DDT. The mixture is quickly heated to 56°C and slowly cooled to room temperature. 1 µl 0.2 M Tris HCl pH 7.5, 0.1 M MgCl₂, 0.01 M DDT, 4 µl 2 mM dNTP, 1 µl 10 mM ATP are added to the mixture, 3 units of T4 DNA ligase (Boehringer, Mannheim) and 2 units of DNA polymerase I (Klenow fragment, Amersham) are added and the polymerization reaction is carried out for 15 hrs at 15°C. Three dilutions of the polymerization mixture are made (1:20, 1:100 and 1:500) in low TE buffer. From each dilution 1 µl and 5 µl are added to 300 µl competent E. coli JM101 cells separately, which have been prepared for transformation by the method described in Example 5.1. The cells are poured on X-gal plates and colonies forming white plaques are obtained. 100 of the white plaques are toothpicked and transferred to LB plates. The bacteria transformed with phage DNA are grown overnight at 37°C and are subsequently transferred to Whatman 541 filters as described in Example 3.1. The filters are washed (Example 3.1.), then prehybridized in 6 x NET, 1 x ss-Denhardt, 0.1 % SDS for 30 min at 67°C. The hybridization is carried out at RT (Example 3.4) for 30 min with 15 pmoles radioactive labelled (Example 3.3.) oligonucleotide 5156 per filter in 6 x SSC. After hybridisation the filters are washed for 4 x 40 sec in 6 x SSC at room temperature and after drying in air exposed 1 hr to Kodak XAR-5 films using an Ilford Screen. The filters are washed a second time for 5 min in 6 x SSC at 72°C (Tm of the mutagenic primer 5156 is 70°C), dried and exposed over night to a Kodak XAR-5 film.

Colonies giving rise to positive signals after the second washing step are picked from the original LB plate, suspended in 1 ml LB medium and heated to 70°C for 20 min. This bacteriophage solution is diluted 1:10, 1:1'000 and 1:100'000 and 10 µl of each dilution is used to transfect 300 µl of exponentially growing E. coli JM101 cells and poured on X-gal plates. 6 plaques of the 1:100000 dilution are toothpicked individually into 1.5 ml 2 x TY containing 15 µl of exponentially growing E. coli JM101 cells and grown at 37°C for 6 hrs. The cultures are centrifuged 5 min in an Eppendorf centrifuge, the supernatants stored at 4°C (phage stock) and the cell pellets are used to do RF preparations according to the method of Birnboim & Doly (17). After restriction analysis with BssHII one mutant phage bearing a new BssHII site in the PLI SalI-EcoRI fragment is chosen for further experiments and designated as (M13mp18-PL(SalI-EcoRI)BssHII/BE5).

### Example 7.3: In-vitro mutagenesis of template M13mp18-PL(SpeI-EcoRI)

The second in-vitro mutagenesis experiment is carried out with single-stranded DNA from phage M13mp18-PL(SpeI-EcoRI), obtained in Example 6. This recombinant phage contains the 1.4 kb SpeI-EcoRI fragment of plasmid pPL29-5 and thus 688 nucleotides of the promoter plus the N-terminal part of the PLI gene. Mutagenesis of this template is carried out exactly as described in Example 7.2. One phage bearing the new BssHII site in the PLI SpeI-EcoRI insert is chosen for four experiments and named respectively M13mp18-PL(SpeI-EcoRI)BssHII/AC5.

### Example 7.4: The construction of plasmid pUBE5

For easier handling the BamHI-EcoRI fragment of phage M13mp8-PL (SalI-EcoRI)BssHII/BE5 obtained as described in Example 7.2, including 130 bp of the promoter region and the N-terminal part of the PLI gene including the newly introduced BssHII site in the signal sequence region, is subcloned into plasmid vector pUN121 (9) as illustrated in Fig. 5 and described in the following.

4 µg RF DNA of phage M13mp8-PL (SalI-EcoRI)BssHII/BE5 is isolated according to Example 5.3. and is digested to completion with restriction endonucleases BamHI and EcoRI (Boehringer, Mannheim). The fragments are separated on a 1 % agarose gel and the gel slice containing the 0.8 kb BamHI-EcoRI fragment, consisting of 117 bp of the promoter and the N-terminal part of the PLI gene is electroeluted as described in Example 2.2. The DNA is extracted with phenol/chloroform and precipitated with ethanol. The DNA pellet is resuspended in 10 µl water (50 ng/µl).

2 µg of pUN121 are digested to completion with BclI and EcoRI (Boehringer, Mannheim). The fragments are separated on a 1 % agarose gel and the 4.0 kb fragment is electroeluted as described in Example 2.2. After phenol/chloroform extraction and ethanol precipitation the DNA is dissolved in 18 µl water (100 ng/µl).

300 ng of the BamHI-EcoRI fragment, comprising the BssHII site, are ligated to 500 ng of BclI/EcoRI cut pUN121 DNA. The ligation is carried out for 4 hrs at 15°C with 400 units T4 DNA ligase (Biolabs) in 20 µl of 50 mM Tris-HCl(pH 7,8) 10 mM MgCl₂, 20 mM dithiothreitol, 1.0 mM ATP. 10 µl of the ligation mix are used to transform 100 µl competent E. coli HB 101 cells (8). The cells are plated on LB plates containing 8 mg/l tetracycline (SIGMA) and incubated overnight at 37°C.

Plasmid preparations are made from 12 tet^{R} colonies according to the method of Birnboim & Doly (17) and one plasmid with the desired restriction pattern shown in Fig. 5 is named pUBE5 and used for further analysis .

### Example 7.5: Introduction of a HgaI restriction site in front of the desulfatohirudin gene of pML310 and the isolation of the 0.22 kB HgaI-BamHI gene fragment

For the introduction of a HgaI restriction site in front of the desulfatohirudin gene (Fig. 6) 8 µg of plasmid pML310 DNA are digested to completion with restriction endonuclease EcoRI. The cleaved DNA is extracted with phenol/ chloroform and precipitated with ethanol. To remove 5' overhanging ends, 4 µg of pML310/EcoRI DNA are digested in 100 µl of 150 mM NaCl, 1 mM ZnSO₄, 30 mM sodium acetate pH 4.6 and 20 U/ml of nuclease S₁ (Sigma) for 45 min at 37°C.

The DNA is extracted with phenol/chloroform and precipitated by ethanol. The DNA (pML310/EcoRI/S₁) is resuspended in 100 µl of 50 mM Tris-HCl pH 8.0 and incubated with 2 units of alkaline phosphatase from calf intestine (CIAP, orthophosphoric-monoesterphosphorylase, EC 3.1.3.1, Boehringer) for one hour at 37°C. The enzyme is inactivated at 65°C for 1.5 hours. The NaCl concentration is adjusted to 150 mM. The dephosphorylated DNA (pML310/EcoRI/S₁/CIAP) is purified by adsorption to a DE52 (Whatman) ionexchange column in a low salt buffer (150 mM NaCl, 10 mM Tris·HCl pH 8.0, 1 mM EDTA). Elution is done with a high salt buffer solution (1.5 M NaCl, 10 mM Tris·HCl pH 8.0, 1 mM EDTA). The DNA is precipitated with ethanol and resuspended in H₂O at a concentration of 0.8 mg/ml.

For the introduction of an HgaI site an oligonucleotide of the formula
5'-AGCGTCGACGCT -3'
is synthesized by the phosphotriester method (21). The sequence of the oligonucleotide is self-complementary containing the recognition site -GACGC- for restriction endonuclease HgaI. Annealing of two single strands leads to a double-stranded DNA linker.

1.2 µg of the synthetic single-stranded oligodeoxynucleotide are phosphorylated in 10 µl of 60 mM Tris·HCl pH 7.5, 10 mM MgCl₂, 5 mM DTT, 30 µCi of [γ-³²P] ATP (3000 Ci·mmol⁻¹, Amersham) and 6 units of T4 polynucleotide kinase (Boehringer) for 30 min at 37°C, followed by a 15 min chase at 37°C in the presence of 0.5 mM ATP. The mixture is further incubated for 10 min at 75°C to inactivate the enzyme. The mixture is cooled to room temperature for annealing.

0.6 µg (170 pmoles) of the ³²P-labelled linker DNA are mixed with 2.4 µg (1.75 pmol ends) of pML310/EcoRI/S₁/CIAP and ligated in 20 µl of 60 mM Tris·HCl pH 7.5, 10 mM MgCl₂, 5 mM DTT, 3.5 mM ATP, 800 units of T4 DNA ligase (Biolabs) for 20 hrs at 15°C. The ligase is inactivated at 85°C for 10 min and the excess of linker molecules is removed by precipitation of the DNA in the presence of 10 mM EDTA pH 7.5, 300 mM sodium acetate pH 6.0 and 0.54 volumes of isopropanol. After 30 min at room temperature the DNA is pelleted resuspended in 45 µl of ligation mixture (specified above) and ligated for 6 hrs at 15°C to form circular DNA.

Aliquots of 1 µl and 3 µl of the ligation mixture are added to 100 µl of calcium-treated, transformation competent E. coli HB101 cells (prepared according to the method of D. Hanahan (10)). The cells are left on ice for 30 min, then incubated for 3 min at 42°C, cooled on ice for 2 min and then incubated for one hour at 37°C in 400 µl of SOC medium. The cells are concentrated in 100 µl of SOC medium each plated on LB agar plates containing 50 µg/ml of ampicillin and are grown overnight at 37°C.

12 transformed amp^{R} colonies are isolated and grown individually in LB medium containing 100 µg/ml of ampicillin. Plasmid DNA is prepared according to the method of D.S. Holmes et al. (22). The presence of the synthetic oligonucleotide linker is confirmed by DNA sequencing using a single stranded DNA fragment as primer which hybridises to the coding strand of hirudin. One clone which contains the linker DNA at the correct position in front of the hirudin gene is referred to as pML310L.

For the isolation of the 0.22 kb HgaI-BamHI desulfatohirudin gene fragment, 40 µg of plasmid pML310L are digested to completion with restriction endonucleases PvuI and BamHI (Boehringer) in 150 µl 0.01 M Tris·HCl pH 7,5, 0.1 M NaCl, 0.01 M MgCl₂, 1 mM 2-mercaptoethanol for 2 hrs at 37°C. The fragments are separated on a 1 % agarose gel and the gel slice containing the 0.84 kb PvuI-BamHI fragment is electroeluted as described in Example 5. After phenol/chloroform extraction and ethanol precipitation the DNA is resuspended in 20 µl HgaI-buffer and digested to completion with restriction endonuclease HgaI (Biolabs). The fragments are separated on a 2 % agarose gel and the gel slice containing the 0.22 kb HgaI- BamHI fragment is eluted as before. After phenol/chloroform extraction and ethanol precipitation the fragment is resuspended in 55 µl sterile water (0.1 pmol/µl).

### Example 7.6: Fusion of the PLI signal sequence and the desulfatohirudin structural gene

To provide a linker in order to ligate the BssHII site of the signal sequence (Example 7.2.) coding region of the PLI gene and the HgaI site of the desulfatohirudin gene (Example 7.5), two oligonucleotides are synthesized as described in Example 3.2. 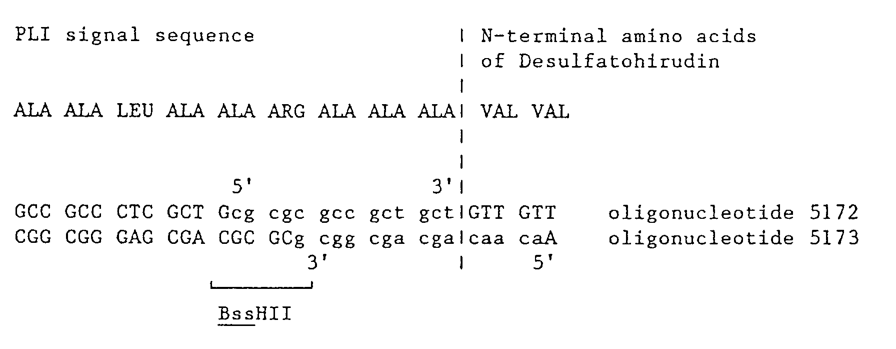
PLI signal sequence | N-terminal amino acids
| of Desulfatohirudin
|
ALA ALA LEU ALA ALA ARG ALA ALA ALA| VAL VAL
|
|
5' 3'|
GCC GCC CTC GCT Gcg cgc gcc gct gct|GTT GTT oligonucleotide 5172
CGG CGG GAG CGA CGC GCg cgg cga cga|caa caA oligonucleotide 5173
3' | 5'
BssHII
(small letters: linker)
300 pmoles of each oligonucleotide are kinased separately with 400 pmoles rATP and 10 units T4 polynucleotide kinase (New England Nuclear) in 15 µl of 50 mM Tris-HCl (pH 7.8), 10 mM MgCl₂, 20 mM dithiothreitol 1.0 mM ATP, 50 µg/ml BSA. Both kinased oligonucleotides are mixed in a 1:1 ratio, heated to 95°C and are slowly cooled down to room temperature for annealing. The annealed linkers are stored at -20°C.

### Example 7.7: The construction of pLHK3

For the construction of a desulfatohirudin expression vector (Fig. 7) comprising the diminished promotor of PLI, 7 µg of pUBE5 DNA (Example 7.4.) are digested to completion with restriction endonuclease BssHII (Boehringer), extracted by phenol/chloroform and precipitated by ethanol. The DNA (1.9 pmol) is resuspended in 10 µl sterile water. 300 pmoles of annealed linker 5172/5173 (Example 7.6.) are ligated to 1.9 pmoles of BssHII-cut pUBE5 with 400 units T4 DNA ligase (Biolabs) in 60 µl of 50 mM Tris-HCl (pH 7.8), 10 mM MgCl₂, 20 mM dithiothreitol 1.0 mM ATP, 50 µg/ml BSA. The ligation is carried out for 15 hrs at 15°C. The ligase is inactivated by heating to 85°C for 10 min. The buffer is adjusted to 0.01 M Tris·HCl pH 8.0, 0,1 M NaCl, 0.005 M MgCl₂, 1 mM 2-mercaptoethanol. 36 units of BamHI (Boehringer) are added and digestion carried out for 2 hrs at 37°C. The fragments are separated on a 1 % agarose gel and the 3.4 kB BamHI-[BssHII]HgaI-linker fragment isolated by electroelution of the gel slice followed by phenol/chloroform extraction and ethanol precipitation. The DNA is resuspended in 14 µl sterile water to a concentration of 0.1 pmol/µl. 0.2 pmols of the 0.22 kb desulphatohirudin HgaI-BamHI fragment are ligated to 0.1 pmol of the digested pUBE5 DNA. Ligation is carried out for 15 hrs at 15°C with 400 units T4 DNA ligase (Biolabs) in 10 µl of 50 mM Tris-HCl (pH 7.8), 10 mM MgCl₂, 20 mM dithiothreitol, 1.0 mM ATP, 50 µg/ml BSA.

2 µl of the ligation mix are used to transform 100 µl of competent E. coli HB101 cells (8), which are subsequently plated on LB dishes containing 50 mg/l ampicilline (SIGMA). The DNA of 12 amp^{R} colonies is prepared by the method of Birnboim & Doly (17) and analysed by restriction analysis. 1 clone is chosen for sequence analysis by the dideoxy chain terminator method of Sanger et al. (23). A clone showing the desired restriction pattern and the correct sequence within the PLI-signal sequence-desulfatohirudin junction is isolated and named pLHK3.

### Example 7.8: The construction of pLHL5

For the construction of the desulfatohirudin expression vector, comprising the complete promoter sequence of PLI gene, 10.3 µg RF DNA of phage M13mp18-PL(SpeI-EcoRI)BssHII/AC5 (Example 3.3.) are digested to completion with restriction endonuclease BssHII (Boehringer), extracted by phenol/chloroform, precipitated by ethanol and resuspended in 10 µl sterile water to a concentration of 1.9 pmoles. 300 pmoles of annealed linker 5172/5176 (see above) are ligated to 1.9 pmoles of the BssHII-cut phage DNA as described in Example 7.7. After inactivation of the T4 DNA ligase the buffer is adjusted to 0.01 M Tris-HCl, pH 7.6, 0.05 M NaCl, 0.01 M MgCl₂, 0,014 M DTT. 36 units of restriction endonuclease HindIII (Boehringer) are added and digestion carried out for 2 hrs at 37°C. The fragments are separated on a 1 % agarose gel and the 1.4 kb HindIII-[BssHII]HgaI-linker fragment is isolated by electroelution as described in Example 2.2. The fragment is resuspended in 15 µl sterile water leading to a concentration of 0.1 pmol/µl.

3 µg of plasmid pBR322 (24) are digested to completion with restriction endonucleases BamHI and HindIII (Boehringer, Mannheim) in 20 µl 0.01 M Tris·HCL pH 7.5, 0.1 M NaCl, 0.01 M MgCl₂, 1 mM 2-mercaptoethanol and the fragments are separated on a 1 % agarose gel. The large 4.15 kb HindIII-BamHI fragment is eluted as described in Example 2.2. and resuspended in 8 µl sterile water (0.1 pmol/µl).

0.2 pmoles of the 0.2 kb HgaI-BamHI fragment of desulphatohirudin and 0.2 pmoles of the 1.4 kb PLI promoter-signal sequence HindIII-[BssHII]HgaI-linker fragment are ligated to 0.1 pmol of HindIII/BamHI-cut pBR322 for 15 hrs at 15°C. The ligation is carried out with 400 units T4 DNA ligase (Biolabs) in 10 µl of 50 mM Tris-HCl (pH 7.8), 10 mM MgCl₂, 20 mM dithiothreitol, 1.0 mM ATP, 50 µg/ml BSA. 1 µl of the ligation mix is used to transfarm 100 µl competent E. coli HB101 cells (8). 12 amp^{R} colonies are isolated and the DNA analyzed as described in Example 7.7. One clone is chosen for further experiments and named pLHL5 (Fig 8).

### Example 7.9: The construction of pLHLT7

To provide a terminator region to the PLI-desulfatohirudin expression System, the 0.7 kb PstI-NheI fragment of plasmid pPL35-5 is fused 3' to the desulphatohirudin gene as illustrated in Fig. 9.

10 µg of pPL35-5 are digested to completion with PstI (Boehringer). The DNA is phenol/chloroform extracted and precipitated by ethanol. The fragments are resuspended in 0.033 M tris-acetate pH 7.9, 0.066 M potassium-acetate, 0.01 M magnesium-acetate, 0.5 mM DTT and 100 ng/ml BSA. 10 units T4 DNA polymerase (Boehringer) are added and the reaction carried out for 180 seconds at 37°C. The reaction mix is put an ice, 20 nmoles of dATP, dCTP, dGTP, dTTP each are added, the buffer adjusted to the above conditions and the reaction carried on for 35 min at 37°C. After phenol/chloroform extraction and ethanol precipitation the DNA is resuspended in 10 µl sterile water (1.9 pmoles = 11.4 pmoles blunt ends). 900 pmoles of kinased and annealed (Example 7.6.) BamHI-linkers (Biolabs) are added and the ligation is carried out for 15 hrs at 15°C with 400 units T4 DNA ligase (Biolabs) in 60 µl 50 mM Tris-HCl (pH 7.8), 10 mM MgCl₂, 20 mM dithiothreitol 1.0 mM ATP, 50 µg/ml BSA. After inactivation of the ligase by heating to 85°C for 10 min, the buffer is adjusted to 0.01 M Tris-HCl pH 8.0, 0.1 M NaCl, 5 mM MgCl₂, 1 mM 2-mercaptoethanol. 20 units restriction endonuclease NheI (Biolabs) are added and digestion is done for 2 hrs at 37°C. The fragments are separated on a 1.2 % agarose gel and the 0.7 kb NheI-[PstI]BamHI fragment is isolated by electroelution as described in Example 2.2. The fragment is resuspended in 15 µl sterile water to give a concentration of 0.1 pmol/µl.

A ligation is set up with 0.2 pmoles 0.7 kb NheI-[PstI]BamHI terminator fragment, 0.2 pmoles 1.4 kb HindIII-[BssHII]HgaI promoter fragment (Example 7.4.), 0.2 pmoles 0.2 kb HgaI-BamHI desulfatohirudin fragment (Example 7.5.) and 0.1 pmoles pUC18 vector (25) linearized by HindIII and XbaI. The reaction is carried out with 400 units T4 DNA ligase (Biolabs) for 15 hrs at 15°C in 10 µl of 50 mM Tris-HCl (pH 7.8), 10 mM MgCl₂, 20 mM dithiothreitol 1.0 mM ATP, 50 µg/ml BSA. 2 µl of the ligation are used to transform E. coli HB101 cells. 12 amp^{R} transformants are analyzed as described in Example 7.6. and the plasmid showing the correct fusion sequences referred to as pLHLT7.

### Example 8: Construction of a cotransformation system for A. niger

### Example 8.1: Preparation of pCG59D7 containing the pyrA gene of Aspergillus niger

300 ng of a 1.1 kb HindIII fragment of pDJB2 (26) bearing part of the N. crassa pyr4 gene are radioactively labelled by nicktranslation according to Maniatis et al., (8). The filter replicas of the gene library (Example 3.1.) are wetted in 6 x NET and prehybridized in 6 x NET, 1 x ss-Denhardt, 0.1 % SDS for 4 hrs at 50°C. Nick-translated HindIII fragment is added (300 ng/80 filters) and hybridization carried out for 15 hrs at 50°C. After hybridization the filters are washed 1 x for 5 min in 4 x SSC, 0.1 % SDS at 50°C. After drying in air the filters are exposed to KODAK X-Omat SO282 films for 3 days. One strongly hybridizing colony, named Escherichia coli BJ5183/pCG59D7 (short 59D7) is isolated and a large plasmid preparation made thereof (14). The plasmid is referred to as pCG59D7 and is used for the transformation in Example 9.2.

### Example 8.2: Mutation of A. niger strain N 756 and isolation of mutants auxotrophic for uridine

Selection for mutants of A. niger strain N 756 specifically lacking orotidine-5'-phosphate decarboxylase activity, can be achieved by positive selection against the toxic analogue fluoroorotic acid (27) in the presence of uridine. 3 x 10⁶ conidial spores of A. niger strain N 756 are plated onto 10 ml of minimal medium plates supplemented with 1 g/l arginine and 50 mg/l uridine. The spares are submitted to short-wave UV-irradiation (2600 Å), at a dosage which results in 0.5 % surviving colonies. After 2 days of incubation at 28°C, when the outgrowing mycelia is slightly visible, 10 mg of fluoro-orotic acid are added to each plate and incubation continued for another 2-3 days. Fluoro-orotic acid resistant colonies appear as heavily growing and sporulating colonies on a background growth of whitish sensitive mycelia. From about 40'000 survivors of mutagenic treatment 8 mutants are isolated. 2 of them which are resistant to fluoro-orotic acid without uridine requirement are not followed further. 6 of them have a uridine auxotrophy. Heterocaryons are made by growing a mixture of conidial spores of two fluoro-acid resistant mutants each on complete medium overnight. Blacks of mycelia are transferred to minimal medium. Mutants complementary to each other in their mutation show outgrowth of prototrophic heterocaryotic mycelia at the edges, strains with a mutation in the same allele do not. The 6 uridine-requiring mutants from strain N 756 fall, as in S. cerevisiae (27), into 2 complementation groups. One of each - An 8 and An 10 - is used for transformation.

### Example 8.3: Preparation of protoplasts and transformation of uridine-mutants An 8 and An 10 of A. niger N 756

Conidial spores of mutants An 8 and An 10 are grown separately on slants for 4 - 5 days at 28°C in complete medium. 2 x 10⁸ conidiospores of An 8 and An 10 are used to inoculate separately 200 ml minimal medium supplemented with 1 g/l arginine and uridine (Example 8.2). After 20 hrs growth at 28°C and 180 rpm. the mycelium is harvested by filtration through Miracloth, washed twice with 10 ml 0.8M KCl 50 mM CaCl₂ and resuspended in 20 µl 0.8M KCl, 50 mM CaCl₂, 0.5 mg/ml Novozym 234 (Novo Industries). The mixture is incubated in a shaking waterbath (30°C, 50 rpm.) until maximum protoplast release can be detected microscopically (90 - 120 min). The protoplast suspension is filtrated through a glass wool plug in a funnel to remove mycelial debris. The protoplasts are pelleted by mild centrifugation (10 min, 2000 r.p.m) at room temperature and washed twice with 10 ml 0.8M KCl 50 mM CaCl₂. The protoplasts are finally resuspended in 200-500 µl 0.8M KCl, 50 mM CaCl₂ to give a concentration of 1 x 10⁸/ml.

For transformation 200 µl aliquots of protoplast suspensions (An 8 and An 10) are incubated separately together with solutions consisting of 10 µg/20 µl pCG59D7 DNA, 50 µl PCT, (10 mM Tris-HCl pH 7.5, 50 mM CaCl₂, 25 % PEG 6000). The incubation mixtures are kept on ice for 20 min, another 2.0 ml of PCT are added and the mixtures incubated for further 5 min at room temperature. 4 ml 0.8M KCl, 50 mM CaCl₂ are added and 1 ml aliquots of these final transformation solutions are mixed with liquefied minimal agar medium (MM + 1 g/l arginine + 10 g/l Bacto-Agar (Difco)) stabilized with 0.8M KCl. The mixtures are immediately poured on agar plates of the same medium and incubated at 28°C.

After 3 days of growth at 28°C, stable transformants appear as vigorously growing and sporulating colonies on a background growth of many hundred small presumably abortive transformants.

Transformation with pCG59D7 is only successful in mutant An 8, not in An 10. An 8 is therefore considered to lack the ornithine-5'-phospate decarboxylase activity, which is complemented by the full-length gene-product of selection plasmid pCG59D7. In An 8 about 20-30 transformants are obtained per µg pCG59D7.

Ten transformants of An 8 are picked, subcultured on minimal medium and the DNA is isolated and analysed for the presence of pCG59D7 sequences. DNA of the transformants is isolated after powdering of the frozen mycelia in liquid nitrogen according to a published procedure (7). 1 µg DNA of each transformant is digested to completion with Xho I, fractionated by electrophoresis over 1 % agarose gels and blotted to nitrocellulose filters according to Maniatis (8), p. 382-386. The blots are hybridized with [α-³²p]-labelled pUN 121 DNA following standard procedures as described by Maniatis et al. (8), p. 387-389. The various hybridization patterns obtained indicate chromosomal integration of the transforming plasmid pCG59D7 at various locations into the host genome.

### Example 9: Expression of the desulfato-hirudin gene under the control of PLI promoter in A. niger

### Example 9.1: Cotransformation of mutant An 8 with pCG59D7, pLHK3, pLHL5 or pLHLT7

Protoplasts of mutant An 8 are prepared according to Example 8.2 and are cotransformed with 5 µg of selection plasmid pCG59D7 and either 10 µg of plasmid pLHK3 (Example 7.7.), 10 µg of plasmid pLHL5 (Example 7.8.) or 10 µg of plasmid pLHLT7 (Example 7.9.), following the procedure as described in Example 8.2.

Transformed An 8 cells are selected on minimal medium for uridine prototrophy as described in Example 8.2. 50 transformants of each cotransformation experiment are randomly picked and analysed for desulfato-hirudin expression. Desulfato-hirudin activity is tested in a thrombin inhibition assay acording to the chromogenic peptide substrate manufacturer's instructions (Boehringer, Mannheim, FRG).

### Example 9.2: Expression of desulfato-hirudin under control of the PLI promoter in transformants of mutant An 8

Conidial spares from transformants obtained according to Example 8.3. are individually precultured into 50 ml of a preculture medium (Pectin Slow Set L (Unipectin, SA, Redon, France) 3 g/l, NH₄Cl 2 g/l, KH₂PO₄ 0.5 g/l, NaCl 0.5 g/l, Mg₂SO₄ x 7 H₂O 0.5 g/l, Ca₂SO₄ x 2 H₂O 0.5 g/l, pH 7.0). The preculture is incubated for 72 hrs at 250 rpm and 28°C. 10 % of the preculture is used to inoculate 50 ml of main culture medium (Soybean flour 20 g/l, pectin Slow Set 5 g/l). The culture is grown up for 72-96 h (highest rate of pectin lyase production) at 250 rpm and 28°C (referred to as inducing conditions). Transformants of mutant An 8 are also grown under non-inducing conditions in Phytone peptone 10 g/l, glucose 10 g/l instead of main culture medium.

At various times (every 20 hrs) samples are taken, the cells are pelleted by centrifugation and broken by ultrasonic desintegration. Supernatant and cell extracts are both tested far desulfato-hirudin activity as described in Example 9.1. No desulfato-hirudin activity is detected following cotransformation with plasmid pLHK3. pLHL5 and pLHL7 containing the full-length PLI promoter are both able to drive expression of desulfato-hirudin in A. niger mutant An 8. Of the 50 transformants taken from the cotransformation experiments (Example 9.1.) 10 each show desulfato-hirudin activity in the medium.

Expression of desulfatohirudin in mutant An 8 under control of the full-length PLI promoter and making use of the PLI signal peptide is therefore regulated by the inducing substrate pectin and leads to secretion of desulfatohirudin into the medium.

### Deposition of Microorganisms

The following microorganisms were deposited under the Budapest Treaty with Deutsche Sammlung von Mikroorganismen, Grisebachstr. 8, D-3400 Göttingen:

| Microorganisms | Dep. Nr. | Date of Dep. |
|---|---|---|
| Escherichia coli BJ5183/pCG3B11 | DSM 3916 | December 11, 1986 |
| Aspergillus niger An8 | DSM 3917 | December 11, 1986 |
| Escherichia coli BJ5183/pCG59D7 | DSM 3968 | February 2, 1987 |

### References

1) F.E.A. van Houdenhoven. Ph.D. Thesius Agricultural University, Wageningen in Communications Agricultural University Wageningen, The Netherlands 75-13 (1975).
2) Edman P., and Begg G. (1967) Eur. J. Biochem. 1, 80-91.
3) J.M. Vereijken, J. Hofsteenge, H.J. Bak and J.J. Beintema. Eur. J. Biochem. 113, 151 (1980).
4) G. Frank and W. Strubert. Chromatographia 6, 522 (1973).
5) A.M. Crestfield, S. Moore and W.H. Stein. J. Biol. Chem. 238, 622 (1963).
6) Knecht et al., Anal. Biochem. 130, 65 (1983).
7) Yelton M.M., Hamer J.E., Timberlake W.E. (1984), Proc. Natl. Acad. Sci. USA 81, 1470-1474.
8) Maniatis T., Fritsch E.F., Sambrook J., Molecular cloning, a laboratory manual, Cold Spring Harbor Laboratory (1982).
9) Nilsson B., Uhlen M., Josephson S., Gatenbeck S. and Philipson L. (1983). Nucleic Acids Research 11, 8019-8030.
10) Hanahan D., (1983). J. Mol. Biol. 166, 557-580.
11) Gergen J.P., Stern R.H., Wensink P.C. (1979). Nucleic Acids Research 7, 2115-2136.
12) M.H. Caruther, Chemical and Enzymatic Synthesis of Gene Fragments: a laboratory manual, Verlag Chemie (1982)
14) Humphreys G.O., Willshaw G.A., Anderson E.S. (1975). Biochimica et Biophysica Acta, 383, 457-463.
15) Messing J. (1983), Methods in Enzymology 101, 21-78.
16) M13 cloning and sequencing handbook, Amersham International plc. Buckinghamshire, England
17) Birnboim H.C. & Doly J. (1979). Nucleic Acids Res 7, 1513-1523.
18) Boel E., Hansen M.T., Hjort I., Hoegh I., Fiil N.P. (1984). EMBO J. 3, 1581-1585.
19) Mount S.M. (1982). Nucleic Acids Res.10, 459-472.
20) Zoller M.J. & Smith M. (1984). DNA 3, 479-488.
21) Itakura et al., J. Am. Chem. Soc. 103, 706 (1981).
22) Holmes D.S., Quigley M. (1981). Anal Biochem 114, 193-197.
23) Sanger T., Nickler S., Coulson A.R. (1977). Proc. Natl. Acad. Sci. USA 74, 5463-5467.
24) Bolivar F., Rodriguez R.L., Greene P.J., Betlach M.C., Heineker H.L., Boyer H., Crosa J.H., Falkow S. (1977). Gene 2, 95-113.
25) Yanish-Perron C., Vieira J., Messing J. (1984). Submitted to Gene.
26) Ballance D.J. and Turner G. (1985), Gene 36, 321-331
27) Boeke J.D., Lacroute F., Fink G.R. Mol. Gen. Geret. 1984, 197, 345-346
28) Norrander, J. et al., Gene 26, 101-106 (1983)

## Claims

1. Recombinant DNA molecules containing DNA sequences coding for the pectin lyase (PL) expression system or a derivative thereof.

2. A recombinant DNA molecule according to claim 1, comprising the promoter, the signal sequence, and the structural gene with or without the terminator of the PLI expression system.

3. A recombinant DNA molecule according to claim 1, comprising a DNA sequence of the formula I or a derivative thereof.

4. A recombinant DNA molecule according to claim 1, which is pCG3B11.

5. A recombinant DNA molecule according to claim 1, comprising the promoter sequence between nucleotides -1 to -688.

6. A recombinant DNA molecule according to claim 1, comprising the signal sequence between nucleotides 1 and 57.

7. A recombinant DNA molecule according to claim 1, comprising the PLI structural gene between nucleotides 58 and 1366.

8. A recombinant DNA molecule according to claim 1, comprising the PLI structural gene without the introns.

9. A recombinant DNA molecule according to claim 1, comprising the terminator extending between nucleotides 1367 and any one of the nucleotides 1570 up to 2029.

10. A recombinant DNA molecule according to claim 1, comprising a DNA sequence of the formula I which is degenerated.

11. A recombinant DNA molecule according to claim 1, which is a recombinant hybrid vector.

12. A recombinant DNA molecule according to claim 1, which is a hybrid vector comprising the PLI structural gene, or a homologous pectin lyase gene or heterologous structural gene.

13. A recombinant DNA molecule according to claim 1, which is a hybrid vector comprising the marker gene pyrA.

14. A recombinant DNA molecule according to claim 1, which is the hybrid vector pLHL5.

15. A recombinant DNA molecule according to claim 1, which is the hybrid vector pLHLT7.

16. Process for the preparation of a recombinant DNA molecule according to claim 1, comprising culturing a host transformed with a DNA molecule containing a DNA sequence coding for the pectin lyase expression system or a derivative thereof and isolating the desired recombinant DNA molecule or the derivative thereof, or preparing it by an in vitro synthesis.

17. A transformed host containing a recombinant DNA molecule according to claim 1.

18. A transformed host according to claim 17 which is Escherichia coli B35183/pCG3B11 (DSM 3916).

19. A transformed host according to claim 17 which is Aspergillus niger An8 (DSM 3917) transformed with a recombinant DNA molecule according to claim 1 and the selection marker plasmid pCG59D7.

20. A transformed host according to claim 17 which is Aspergillus niger An8 (DSM 3917) transformed with pLHK3, pLHL5 or pLHLT7, and the selection marker plasmid pCG59D7.

21. Method for the preparation of a transformed host according to claim 17, comprising treatment of such host under transforming conditions with a recombinant DNA molecule according to claim 1, optionally together with a selection marker gene and selecting the transformants.

22. Method according to claim 21, wherein Aspergillus niger An8 is cotransformed with a recombinant DNA molecule according to claim 1 and the selection marker plasmid pCG59D7.

23. Use of a recombinant DNA molecule according to claim 1 for the preparation of hybrid vectors which express useful polypeptides.

24. Method for the preparation of a polypeptide, characterised in that a hybrid vector according to claim 11 is expressed in a suitable host and the polypeptide is isolated.

25. Method according to claim 24 for the overproduction of PLI in Aspergillus species comprising cultivating an Aspergillus host transformed with an expression hybrid vector for the expression and secretion of PLI and collecting the PLI from the nutrient medium.

26. The selection marker plasmid pCG59D7.

27. A host containing the plasmid according to claim 26.

28. A host according to claim 27, which is Escherichia coli BJ5183/pCG59D7 (DSM 3968).

29. Aspergillus niger An8 (DSM 3917).

## Claims (Claims for the following Contracting State(s): ES)

1. A process for the preparation of a recombinant DNA molecule containing DNA sequences coding for the pectin lyase (PL) expression system or a derivative thereof, characterized in culturing a host transformed with a DNA molecule containing a DNA sequence coding for the pectin lyase expression system or a derivative thereof and isolating the desired recombinant DNA molecule or the derivative thereof, or preparing it by an in vitro synthesis.

2. A process according to claim 1, characterized in that a recombinant DNA molecule is prepared, comprising the promoter, the signal sequence, and the structural gene with or without the terminator of the PLI expression system.

3. A process according to claim 1, characterized in that a recombinant DNA molecule is prepared, comprising a DNA sequence of the formula I or a derivative thereof.

4. A process according to claim 1, characterized in that a recombinant DNA molecule is prepared, which is pCG3B11.

5. A process according to claim 1, characterized in that a recombinant DNA molecule is prepared, comprising the promoter sequence between nucleotides -1 to -688.

6. A process according to claim 1, characterized in that a recombinant DNA molecule is prepared, comprising the signal sequence between nucleotides 1 and 57.

7. A process according to claim 1, characterized in that a recombinant DNA molecule is prepared, comprising the PLI structural gene between nucleotides 58 and 1366.

8. A process according to claim 1, characterized in that a recombinant DNA molecule is prepared, comprising the PLI structural gene without the introns.

9. A process according to claim 1, characterized in that a recombinant DNA molecule is prepared, comprising the terminator extending between nucleotides 1367 and any one of the nucleotides 1570 up to 2029.

10. A process according to claim 1, characterized in that a recombinant DNA molecule is prepared, comprising a DNA sequence of the formula I which is degenerated.

11. A process according to claim 1, characterized in that a recombinant DNA molecule is prepared, which is a recombinant hybrid vector.

12. A process according to claim 1, characterized in that a recombinant DNA molecule is prepared, which is a hybrid vector comprising the PLI structural gene, or a homologous pectin lyase gene or heterologous structural gene.

13. A process according to claim 1, characterized in that a recombinant DNA molecule is prepared, which is a hybrid vector comprising the marker gene pyrA.

14. A process according to claim 1, characterized in that a recombinant DNA molecule is prepared, which is the hybrid vector pLHL5.

15. A process according to claim 1, characterized in that a recombinant DNA molecule is prepared, which is the hybrid vector pLHLT7.

16. Method for the preparation of a transformed host containing a recombinant DNA molecule according to claim 1, comprising treatment of a host under transforming conditions with a recombinant DNA molecule according to claim 1, optionally together with a selection marker gene and selecting the transformants.

17. Method according to claim 16, wherein Escherichia coli B35183/pCG3B11 (DSM 3916) is prepared.

18. Method according to claim 16, wherein Aspergillus niger An8 (DSM 3917) is transformed with a recombinant DNA molecule according to claim 1 and the selection marker plasmid pCG59D7.

19. Method according to claim 16, wherein Aspergillus niger An8 (DSM 3917) is transformed with pLHK3, pLHL5 or pLHLT7, and the selection marker plasmid pCG59D7.

20. Method for the preparation of a polypeptide, characterised in that a hybrid vector according to claim 11 is expressed in a host transformed therewith and the polypeptide is isolated.

21. Method according to claim 20 for the overproduction of PLI in Aspergillus species comprising cultivating an Aspergillus host transformed with an expression hybrid vector for the expression and secretion of PLI and collecting the PLI from the nutrient medium.

22. Process for the preparation of a recombinant DNA molecule containing the DNA sequence coding for the pyrA gene of Aspergillus niger, characterized in culturing a host transformed with a DNA molecule containing the DNA sequence coding for the pyrA gene and isolating the desired recombinant DNA molecule.

23. A process according to claim 22, wherein the plasmid pCG59D7 containing the pyrA gene is prepared.

24. A method for the preparation of a transformed host containing a recombinant DNA molecule according to claim 22, which comprises treating a host under transforming conditions with said recombinant DNA molecule.

25. A method according to claim 24, wherein Escherichia coli BJ5183/pCG59D7 (DSM 3968) is prepared.

26. A method for the preparation of the pyrA deficient Aspergillus niger An8 (DSM 3917), characterized in that conidiospores of Aspergillus niger N756 are treated under mutating UV-irradiation and colonies surviving in the presence of fluoro-orotic acid and uridine are selected.
